# EUROPEAN PATENT APPLICATION

(11) **EP 1 710 319 A1**
(43) Date of publication of application: **11.10.2006**
(21) Application number: 05075813.5
(22) Date of filing: 08.04.2005
(51) Int. Cl.: C12Q 1/04, G01N 33/50

(54) **Production of unconjugated oligosaccharides by pathogens**

(71) Applicant: Academisch Ziekenhuis Leiden, 2333 ZA Leiden (NL)
(72) Inventor: Deelder, André Martien, 2341 NE Oegstgeest (NL); Hokke, Cornelis Hendrik, 2582 XP Den Haag (NL); Robijn, Marjolein Louise Maria, 2274 GN Voorburg (NL)
(74) Representative: Winckels, Johannes Hubertus F.

(57) **Abstract**

The invention relates to means and methods for analysing a sample for the presence of a pathogen or pathogen related products therein. In one embodiment a method of the invention comprises determining whether said sample comprises pathogen specific unconjugated oligosaccharide.

## Description

The invention relates to the field of oligosaccharide production by living organisms. The invention in particular relates to unconjugated oligosaccharide production by pathogens of animals and plants.

Oligosaccharides display a broad spectrum of structural variety in numerous biological systems and are linked to lipids or proteins, to form so-called glycoconjugates. Glycoconjugates are found inside cells, within cell membranes as well as in extracellular fluids and matrices and play an important role in biological recognition processes.

Glycoconjugates generally comprise one or more mono- or oligosaccharides covalently linked to an aglycone (mostly protein or lipid). Several different types of glycoconjugates commonly found on cells can be discriminated. A *glycoprotein* is a glycoconjugate in which a protein carries one or more oligosaccharide chains covalently attached to their polypeptide backbone, usually via *N-* or *O*-glycosidic linkages. An *N-glycan* is an oligosaccharide that is covalently linked to an asparagine residue of a polypeptide chain within the consensus peptide sequence: Asn-X-Ser/Thr. The *N*-glycans contain a common core structure consisting of two *N-*acetylglucosamine (GlcNAc) residues and three mannose (Man) residues and can be generally divided into three main classes: oligomannosidic-type, complex- type and hybrid-type. An *O-glycan* is linked to a polypeptide via N-acetylgalactosamine (GalNAc) to a serine (Ser) or threonine (Thr) residue and can be extended into a variety of different structural core classes.

*A glycosphingolipid* is a molecule containing a (mono)saccharide linked to a lipid aglycone, wherein the oligosaccharide is usually attached via glucose (Glc) or galactose (Gal) to ceramide. The glycosphingolipids are classified according to different core series. A *glycosylphosphatidylinositol* (GPI) anchor is a membrane anchor consisting of a glycan bridge between phosphatidylinositol and a phosphoethanolamine linked to a protein. A *proteoglycan* is a protein with one or more covalently attached glycosaminoglycan chains. The glycosaminoglycans are linear polysaccharides composed of repeating disaccharide units containing either GlcNAc or GalNAc and uronic acid (glucuronic acid (GlcA) and iduronic acid) (except for keratan sulphate) and covalently attached sulphate groups (except for hyaluronic acid). In contrast to the unique core regions of different classes of glycans (see above), terminal structural sequences are often shared among different classes of glycans.

### Biosynthesis of glycoconjugates

Three major glycosylation pathways can be distinguished, i.e. protein glycosylation routes leading to *N*- or *O*-linked glycans or glycolipids. These processes occur within the endoplasmic reticulum (ER) and the Golgi apparatus. Newly synthesized proteins or lipids originating from the ER are co- or post-translationally decorated with carbohydrate chains at various stages in the process to their final destination. The biosynthesis proceeds by the sequential addition of monosaccharide building blocks. The possible combination of different monosaccharides and linkages results in a large variety of structures. In addition, other modifications of monosaccharides, such as phosphorylation, sulphation, methylation or fatty acylation, increase the diversity of glycans.

### Protein N-glycosylation

The biosynthesis of *N*-glycans starts in the rough ER by the stepwise formation of the precursor- structure Glc₃Man₉GlcNAc₂ linked to the membrane-bound lipid dolichol (Kornfeld and Kornfeld 1985; Schachter 1991; Van den Eijnden 2000). This precursor structure is then transferred en bloc to an Asn residue, within the consensus sequence Asn-X-Ser/Thr (where X can be any amino acid except Pro or Asp), of a newly synthesized polypeptide at the ribosome. Subsequently, the oligosaccharide is extensively trimmed resulting in an oligo-mannosidic type structure. This structure may then be converted into more complex structures by subsequent elongation steps taking place in the medial- and trans-Golgi cisternae. Addition of a GlcNAc residue, followed by the removal of the additional Man residues, and subsequent addition of a GlcNAc residue, results in di-antennary structure. Further processing results in a wide variety of possible structures: di-, tri-, or tetra-antennary oligosaccharides, hybrid or bisected structures, or core-fucosylated structures. Elongation of these branches occurs by the addition of a Gal or GalNAc and can be further processed in the presence of different glycosyltransferases to mature oligosaccharides.

### Protein O-glycosylation

*O*-glycans are linked to Ser or Thr proteins. The formation of *O-*linked glycans takes place by sequential addition of monosaccharides in the smooth ER and the Golgi apparatus (Schachter and Brockhausen 1989; Van den Eijnden 2000; Schachter 2000). In contrast to *N*-glycosylation, *O-*glycosylation is not initiated by the *en bloc* transfer of a lipid linked precursor structure, but by the transfer of GalNAc to Thr or Ser on the polypeptide chain. The core structures can be extended by the addition of Gal, GaINAc, GlcNAc, fucose (Fuc) and *N*-acetylneuraminic acid (NeuAc) in different ways leading to a great variety of *O*-glycans.

### Glycosphingolipid biosynthesis

After the synthesis of ceramide, it is glucosylated by a ceramide-specific glucosyltransferase or galactosylated by a specific galactosyltransferase. In mammalian cells, the next addition to the glucosylceramide is always a β-linked galactose residue. Each of these basic units can be further elongated by the stepwise addition of monosaccharide residues. In addition, as with *N*- and *O*- glycans, these outer monosaccharide residues are targets for additional modifications such as *O*- sulphation. Many of these glycosphingolipids can be categorized according to their specific core structures (Basu *et al*. 2000; Varki 1999b).

### Glycosaminoglycans biosynthesis

The biosynthesis of the glycosaminoglycans, with the exception of hyaluronic acid and keratan sulphate, starts with building of the tetrasaccharide GlcAβ1-3Galβ1-3Galβ1-4Xylβ-Ser, which is further extended with the various repeating disaccharides resulting in the different types of glycosaminoglycans (Esko 1999). After formation of the polysaccharide chain numerous modifications occur, such as sulphation and acetylation.

### GPI anchor biosynthesis

The biosynthesis of GPI anchors occurs in two major steps. The first step is the preassembly of the donor GPI in the ER. This is a stepwise process in which sugars and phosphoethanolamine are sequentially added to a phosphatidylinositol, resulting in a complete GPI precursor. Then the GPI precursor is transferred to the newly synthesized polypeptide (Morita *et* al. 2000; Hart 1999).

### Glycosyltransferases

The biosynthesis of oligosaccharide chains is mediated by the sequential action of glycosyltransferases (reviewed in Van den Eijnden (2000)). These enzymes typically catalyse the following general reaction: nucleoside-P-P-sugar + acceptor -> sugar-acceptor + nucleoside-P-P

In this reaction nucleotide-sugars are the donor substrates; the sugar-acceptor product of one reaction is the acceptor substrate in the next. Glycosyltransferases are highly specific for the sugar they transfer and the linkage they establish. They are, furthermore, generally specific for the accepting sugar and the linkage by which this sugar is linked to or is substituted by another sugar residue. Because of these properties glycosyltransferases can be used for the *in vitro* synthesis of specific and unique oligosaccharide chains in high yields. Glycosyltransferases can be extracted in active form for example from invertebrates, cell cultures, blood or milk, or can be produced as recombinant enzymes, and be used for the *in vitro* synthesis of different glycan epitopes. The glycosyltransferases are the most important regulators of the glycosylation pathways which happen in a cell-, tissue-, species- and stage-specific way. By varying the expression levels of glycosyltransferases in cells, the glycosylation pattern of a certain glycoprotein or glycolipid can be altered. For example, altered glycosylation is a universal feature of tumour cells, which is attended by changes in glycosyltransferase activity. These changes can lead to metastatic phenotypes. In addition, endo- or exo-glycosidases that specifically cleave glycosidic linkages are expressed by the cell and together with the glycosyltransferases they ultimately determine the structure and form of glycoconjugates and oligosaccharides.

### Functions of glycoconjugates

As might be anticipated from the complex nature of oligosaccharides and their great variety found in different organisms and on different cell types within one organism, the biological roles of glycans are quite varied. Given the enormous diversity of glycans in nature, the biological roles of glycans span the spectrum from those that are trivial to those that are crucial for the development, growth, function or survival of an organism. The functions can be divided roughly into two groups. One group is based on structural and modulatory properties of glycans and the other relies on specific recognition of glycan structures by other molecules, i.e. lectins.

### Structural and modulatory roles of glycans

Glycans have many protective, organizational and barrier functions. The surfaces of most types of cells are covered with a dense coating of sugars, the so-called glycocalyx and can represent a substantial physical barrier. The glycans attached to matrix molecules such as proteoglycans are important for the maintenance of tissue structure, porosity and integrity. In addition, glycans can have an indirect role in facilitating and/or in maintaining the correct folding of a protein (in the ER) or in protecting a protein against proteolytic degradation.

Furthermore, glycosylation can modulate the interaction of proteins with one-another. For example, glycosylation can mediate an on-off switching effect (such as for the hormone β-human chorionic gonadotrophin) (Willey 1999) or tune the primary function of a protein (effect of heparin/heparin sulphate chains on the action of the anti-coagulant antithrombin III) (Kindness *et al*. 1980).

### Glycans as recognizing molecules

Lectins are carbohydrate-binding proteins that specifically recognize diverse sugar structures and mediate a variety of biological processes, such as cell-cell, host-pathogen interactions, serum glycoprotein turn-over and innate immune responses. Lectins can be distinguished into C- type lectins, P-type lectins, S-type lectins and I-type lectins. C-type lectins (e.g. selectins, collectins, lymphocyte lectins) are involved in many functions of the immune system, such as inflammation and immunity to tumours or virally infected cells and in innate immunity. P-type lectins, such as mannose-6-phosphate receptors, are involved in the targeting of newly synthesized lysosomal enzymes to their final destination in the lysosomes. The proposed biological functions of the S-type lectins (more recently termed galectins) include roles in cell- cell adhesion, cell-matrix adhesion, direct effects on cell growth and viability, recognition of pathogens and potential intracellular functions in regulation metabolism. Their expression in many types of tumour cells has led to the hypothesis that galectins may also be involved in tumorigenesis and metastasis. Due to altered glycosylation on tumour cells, these cells could not be recognized by lectins and this effect enhances the invasion and the metastatic cascade of carcinoma cells. Finally, I-type lectins are a family of carbohydrate-binding proteins within the immunoglobulin superfamily. Amongst the I-type lectins, there is a group of sialic-acid binding lectins, the so-called siglecs. So far, siglecs have been shown to be involved in hemopoiesis, neural development and immunity (e.g. regulation of B-cell antigen receptor signalling).

Carbohydrate-carbohydrate interactions may also play a specific role in cell-cell interactions and adhesion. For example, the compaction of the mouse embryo at the morula stage seems to be due to a Lewis^{x}-Lewis^{x} interaction (Pincet *et al.* 2001). Glycans can also be a specific ligand for viruses, bacteria, and parasites and for plant and bacterial toxins (Varki 1999a). In addition, they can serve as antigens for autoimmune and alloimmune reactions. From the above it is clear that oligosaccharides are often produced by organisms in the context of a conjugate. However, unconjugated oligosaccharides are also produced. It has been described that (mostly small) free oligosaccharides are present in urine. Oligosaccharides that can be found in urine are diverse. Oligosaccharides such as lactose, sialyllactose, or glucose tetrasaccharide (Glc₄), or oligosaccharides bearing A, B, or O blood group determinants are commonly found in non-pathological urine samples. Increased oligosacchariduria is seen in the urine of lactating or pregnant women, breast milk-fed neonates, or preterm infants fed with milk fortified with Glc polymers. Abnormal excretion of oligosaccharides is found in glycoprotein degradation disorders and in some glycogen storage diseases. In these lysosomal diseases, analysis of urinary oligosaccharides is used as a diagnostic aid. For example in the case of α-mannosidosis, the deficiency of α-mannosidase leads to impaired lysosomal degradation of glycoprotein N-glycans. Normally these are degraded to monosaccharides by the sequential action of glycosidases. If glycosidases in this sequence are deficient, larger fragments of glycoconjugate glycans remain that can be traced in urine. Also in milk many free oligosaccharides, such as lactose, sialyllactose, LNT, LNnT and fucosylated versions thereof are present.

Conjugated or free oligosaccharides typically consist of hexoses, pentoses, deoxyhexoses, (*N*-acetyl)hexosamines and sialic acids that are linked to each other and to the carrier of the oligosaccharide. Considering the variety of different monosaccharides and the variety in linkage between them, the variability in the carbohydrate portion of the glycoconjugate can be enormous. Considering the importance of glycoconjugates it is not surprising that pathogens have found ways to use glyconjugates to their own advantage. The other way around, many pathogens produce glycoconjugates that are recognized by host lectins, and that are particularly important in the immunological interaction with the host. For example, *Schistosoma* produce a plethora of glycoconjugate structures that on the one hand are identical to that of the host or other helminth species, but on the other hand contain many highly unusual species-specific structures. Each helminth species expresses its own species-specific set of glycoconjugates (Dell *et al*., 1999; Hokke and Deelder 2001; Nyame et al., (2004). In addition, polysaccharides from different bacteria contain different glycan elements specific for each bacterial species or strain. For example, group B Streptococcus consists of at least nine serotypes distinguishable on the basis of their capsular polysaccharide antigen (Paoletti and Kasper, 2003).

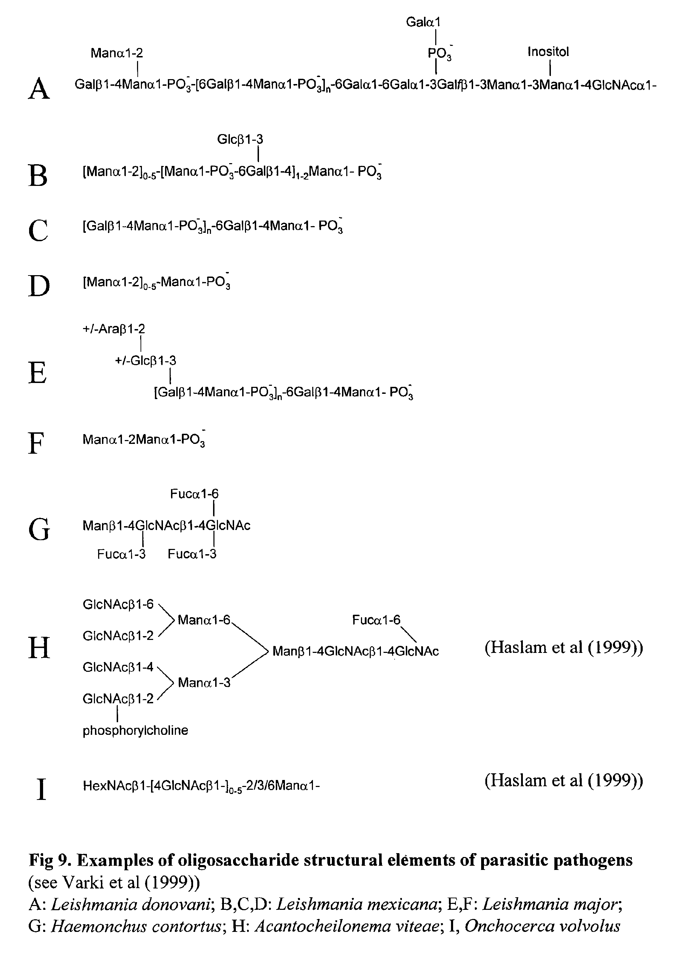

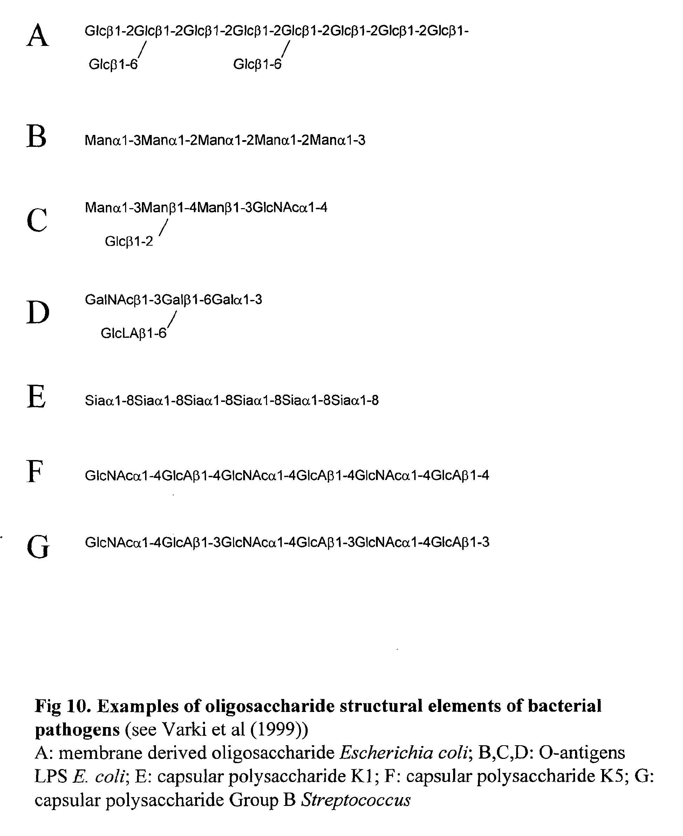

In the present invention it has surprisingly been found that pathogens produce unconjugated oligosaccharides. These so-called free oligosaccharides can be detected specifically against the background of biological material obtained from animals. The present invention therefore provides a method for determining whether a sample comprises oligosaccharide produced by a pathogen said method comprising determining whether said sample comprises unconjugated oligosaccharide. In a preferred embodiment, the invention provides a method for analysing a sample comprising determining whether said sample comprises pathogen specific unconjugated oligosaccharide. A pathogen specific unconjugated oligosaccharide is an unconjugated oligosaccharide that is produced by or in response to said pathogen. The pathogen specific unconjugated oligosaccharide can be discriminated in kind and/or in amount from other unconjugated oligosaccharide present in said sample. Pathogen specific unconjugated oligosaccharide can be produced directly by said pathogen or by cells of the host, in response to the presence of the pathogen in the host. Said pathogen specific unconjugated oligosaccharide preferably comprises a structure of figure 9, figure 10 or table 1 or a functional part, derivative and/or analogue thereof. Many pathogen specific conjugated oligosaccharides (or specific parts thereof) can be produced as pathogen specific unconjugated oligosaccharide. Non-limiting examples of such pathogen specific conjugated oligosaccharides are described in Hokke and Deelder 2001, Dell et al 1999; Benz en Schmidt 2002, Upreti et al 2003. A functional part of said unconjugated oligosaccharide is preferably a breakdown product of a structure as described in figure 9, figure 10 or table 1. In a preferred embodiment said functional part comprises a Fucα1-2Fucα1-3GlcNAc/GalNAc element. A derivative of a structure of figure 9, figure 10 or table 1 is a structure comprising one or more modifications of the structure as presented. Such modifications are for instance the result of commonly observed microheterogeneity, or due to the presence of additional monosaccharides or aglycone substituents such as methyl groups, phosphates, acyl chains etc. Analogues of a structure as presented in figure 9, 10 or table 1 are structures having the same side chains in kind not necessarily in amount but a different backbone.

Unconjugated oligosaccharide is not linked to a protein or a lipid. An oligosaccharide of the invention is a molecule comprising a between 1 and 40 and preferably between 3 and 30 monosaccharide units. A monosaccharide is a simple sugar that does not hydrolyse to give smaller sugars. A monosaccharide (when coupled to another monosaccharide or aglycone) typically has a fixed 5- or 6- membered ring structure of a single oxygen atom and multiple carbon atoms, substituted with hydroxyl groups or numerous possible substituents. The non-coupled reducing monosaccharide exists as an equilibrium between the different possible ring-forms and a linear non-ring form. In its most basic form, the hexose type monosaccharide has the general formula (CH₂O)ₙ, hence the general name' carbohydrate'. An unconjugated oligosaccharide of the invention has typically a mass of between 500 and 6000 Dalton. Preferably, said unconjugated oligosaccharide has a mass between 1000 and 4000 Dalton.

The sample can be any sample suspected of containing unconjugated oligosaccharide produced by said pathogen. In a preferred embodiment the sample is obtained from fluid wherein the pathogen is cultured. In this case unconjugated oligosaccharides produced by the pathogen may be analysed. For instance, for the purpose of determining unconjugated oligosaccharide production in the absence of a host for the pathogen. In another preferred embodiment the sample is obtained from an animal or a plant. In this embodiment, unconjugated oligosaccharide production of the pathogen, or as the result of the presence of the pathogen in the host can be determined when the pathogen is present in or on an animal or plant. Said method may be used for detection of the pathogen in or on the animal or plant. Alternatively, the method is used to analyse the interaction of the pathogen with the animal or plant. This is useful, for instance, to determine whether and to what extent counter measures of the plant or animal affect the pathogen. Thus in a preferred embodiment the sample comprises fluid obtained from an animal or plant. The fluid can be a fluid present in the animal or plant, or a fluid obtained by processing of (a part) of an animal or plant. For instance, muscle, organ tissue or leaves may be extracted. Liquid obtained from the extraction can subsequently be used in the sample.

In a preferred embodiment the sample fluid comprises urine, blood, saliva, lymph fluid, cerebral spine fluid or synovial fluid. Preferably, the sample fluid comprises urine. Urine is easy to collect and sample volumes can be large without affecting the biological status of the animal. Larger sample volumes of course, increase the sensitivity of a method of the invention. Sample volumes of at least 1 and more preferably at least 100 ml are preferred.

Unconjugated oligosaccharide can be determined as a group, for instance by subtracting conjugated oligosaccharide from total oligosaccharide in said sample or by selective monitoring or extraction of the unconjugated oligosaccharides. In a preferred embodiment, at least one purification step is performed prior to the analysis. Purification is not required but typically increases sensitivity, and improves robustness of a method of the invention. In a preferred embodiment, at least some saccharides are separated from other constituents of the sample by incubating the sample with a binding molecule that is specific for a carbohydrate structure present on an oligosaccharide. As binding molecules are typically specific for a subset of structures present on saccharides, the incubation results in binding of a subset of saccharides present in the sample. By separating bound from unbound material the (saccharide) complexity is reduced. Unconjugated oligosaccharide may be determined in the unbound fraction or the bound fraction. Preferably, the bound fraction is analysed for unconjugated oligosaccharide. Thus in a preferred embodiment, a method of the invention further comprises determining whether said binding molecule comprises unconjugated oligosaccharide.

Saccharides can have many different structures. Many different binding molecules have been generated that specifically recognize such structures. Binding molecules may recognize one or more structures present on an oligosaccharide. Typically a binding molecule is specific for only one structural element on an oligosaccharide. However, since the recognized structure is only part of the oligosaccharide, the recognized oligosaccharides typically differ in parts that are not recognized by the binding molecule. Thus a binding molecule typically binds a collection of different saccharides that share a common structure.

A structure that is present on an oligosaccharide can also be present on a polysaccharide or a saccharide part of a glycoconjugate. Thus, after incubation with the sample, the binding fraction typically contains unconjugated oligosaccharide, polysaccharide and/or glycoconjugate. Unconjugated oligosaccharide is therefore typically determined in a subsequent analysis wherein oligosaccharide is further separated from other saccharide containing molecules, or selectively visualised. Thus in a preferred embodiment a method of the invention further comprises subjection of the fraction suspected of containing unconjugated oligosaccharide to a separation step and detecting separated unconjugated oligosaccharide. The separation step preferably comprises a separation on the basis of size, mass or polarity. To this end, a chromatographic system can be used employing graphitised carbon, ion-exchange, normal phase, reverse phase columns and other systems known to be suitable for the separation of various classes of oligosaccharides. Alternatively, capillary electrophoresis can be carried out. The fractionated oligosaccharides are detected on-line, either by mass spectrometry, UV or by fluorescence (after labelling of the free oligosaccharides prior to fractionation using suitable reagents such as 2-aminobenzamide or APTS). Alternatively, fractions are collected after chromatographic or electrophoretic separation or directly on a MALDI-target or in any of collector and the contents of the fractions is characterised by spectrometric or spectroscopic methods off-line. Chromatographic or electrophoretic methods including fluorescence detection of the oligosaccharide profile are ideally suited for the purpose of a sensitive and robust assay.

In a preferred embodiment a method of the invention further comprises separating at least two unconjugated by means of mass spectrometry.

In the present invention it has been found that pathogens produce unconjugated oligosaccharides. The pathogen is preferably a microbe or a parasite. The pathogen can produce a particular unconjugated oligosaccharide together with a conjugated form of the particular oligosaccharide. However, the unconjugated oligosaccharide does not need to have a conjugated counterpart. It is also possible that the unconjugated oligosaccharide is produced by processing of conjugated oligosaccharide. In a particularly preferred embodiment said pathogen is a parasite, preferably a worm parasite. Parasites typically mature via several stages and not all stages are very easily detected and/or quantified via other means. In the present invention it has been found that respective stages produce characteristic unconjugated oligosaccharides. The various stages can therefore easily be detected and/or quantified by analysing at least one unconjugated oligosaccharide produced by the pathogen. Quantifying of one or more unconjugated oligosaccharides that are specific for a particular stage provides information on the number and/or quantity of a certain stage of parasite. In a particularly preferred embodiment the parasite is a helminth. Helminths belonging to the genus *Schistosoma* are preferred. In a preferred embodiment helminth egg-specific unconjugated oligosaccharides are detected. Below a detailed description of the pathology and characteristics of *Schistosoma* infections is described. Though the helminth genus *Schistosoma* is preferred in the present invention, the invention is not limited to this genus.

The degree of morbidity caused by schistosomiasis depends on the species, the intensity of infection and environmental and host-related factors. The majority of the 200 million infections show relatively mild symptoms and approximately 20 million people suffer from severe consequences of infection. Annually, 200,000 deaths are estimated to be associated with schistosomiasis (Van der Werf *et al*. 2002). This mortality is mainly due to liver fibrosis and portal hypertension in the case of intestinal schistosomiasis or to bladder cancer or renal failure in the case of urinary schistosomiasis. The disease weakens infected individuals and therefore has serious consequences on the socio-economic development of tropical and subtropical areas (Gryseels 1989).

Three distinct syndromes caused by schistosomiasis have been described: cercarial dermatitis, acute schistosomiasis and chronic schistosomiasis.

### Cercarial dermatitis:

The so-called cercarial dermatitis or swimmer's itch is caused by penetration of the cercariae of schistosomes into human skin, which may provoke an acute inflammatory response. The dermatological response in the human host is variable and is dependent on the degree of hypersensitivity induced by previous exposure. Initial exposure to cercariae produces only mild, transient reactions that often pass unnoticed. The diagnosis is difficult and treatment is usually not needed.

In non-endemic areas, cercariae from avian blood flukes that are not able to complete their life cycles in man, can often penetrate human skin and transform to schistosomula. They persist for up to 10 days post exposure and can give rise to an allergic-type immediate type hypersensitivity reaction (Horak and Kolarova 2000). Swimmer's itch is prevalent in many parts of both the developed and developing countries; recently there was a new outburst as far north as Iceland (Kolarova *et al*. 1999).

### Acute schistosomaasis:

Three to nine weeks after infection, an allergic reaction characterized by fever, headache, cough, loss of appetite, abdominal pain, diarrhoea and often eosinophilia can develop; this syndrome is called Katayama fever or acute schistosomiasis. The majority of cases begin with the deposition of eggs in the host tissue. At this stage the clinical diagnosis is very difficult because in general it is still too early to reliably demonstrate eggs in the excreta (Polderman and De Caluwe 1989; Visser *et al*. 1995).

### Chronic schistosomiasis:

Chronic schistosomiasis may occur even without any recognizable symptoms and can last for decades. Morbidity arises slowly and is accompanied by pathological changes in affected organs. Schistosome eggs that become lodged within the host tissues are the major cause of pathology. Antigens secreted by miracidia through microscopic pores within the eggshell induce a chronic granulomatous inflammatory response, which may eventually lead to fibrosis of the liver, or urinary bladder, depending on the species. The active acute granulomatous response gradually becomes downregulated into a chronic phase. Initial symptoms of chronic schistosomiasis are diarrhoea, dysentery, abdominal pain, lack of appetite, weight loss, proteinuria and bloody stool or haematuria. Severe infections can result in portal hypertension, hepatomegaly, splenomegaly, ascites, bleeding varices (intestinal schistosomiasis), or obstruction of the urinary tract (urinary schistosomiasis).

### Treatment, control and vaccination

Currently, Praziquantel is the common platyhelminthicide used to treat schistosomiasis. A single oral dose of 40 mg/kg of Praziquantel is generally sufficient to give cure rates of between 60- 90% and reductions of 90-95% in the average number of excreted eggs.

Recent studies have shown that the anti-malaria drug Artemether destroys juvenile forms of schistosomes *(S. haematobium, S. japonicum* and S. *mansoni)* (Shuhua *et al.* 2000a; Shuhua *et al.* 2000c; Xiao *et al.* 1994). Praziquantel and Artemether affect schistosomes at different developmental stages and work surprisingly well together in this respect (Shuhua *et al.* 2000b). Whether the two drugs can actually be given simultaneously and whether there is any pharmacological interaction remains to be investigated. Until now, there is no malaria resistance to Arthemeter, however, by increasing the use of this drug, drug-resistance may be induced. Therefore, the use of this drug as treatment for schistosomiasis is only possible in a malaria-free area, whereas malaria is endemic in 75% of the schisto-endemic areas.

Since chemotherapy does not prevent re-infection, regular treatment in endemic areas is needed due to continuous exposure. The control strategy of WHO is to reduce the morbidity caused by schistosomiasis rather than to halt or block transmission entirely. Controlling morbidity with drug treatment is a feasible and effective strategy. Other major interventions are health education and the provision of safe water (Engels *et at*. 2002). The development of a vaccine that could offer at least partial immunity would be an important step forward in the control of schistosomiasis. The possibility of acquired immunity is supported by the fact that the prevalence and intensity of infection decreases with age. Relatively little research has been carried out to identify suitable vaccine candidates. Vaccine research is either focused on reducing prevalence directly by blocking the establishment of the worms or on the reduction of morbidity by focusing on an anti-fecundity effect by blocking the egg laying process of the worms. Vaccinations of experimental animals both under laboratory and field conditions have been performed using cercariae attenuated by ultraviolet (UV) or gamma irradiation (Kamiya *et at.* 1993; Shi *et at*. 1990; Smith and Clegg 1984; Stek *et at.* 1981). The results were promising but are not applicable to human vaccination, as attenuated vaccines are considered neither safe nor practicable. The attenuated vaccine consists of living organisms, so there is no control where these parasites terminate in the human body. An additional drawback is the supply and production of cercariae.

Other studies have been performed to identify relevant schistosome antigens that may be involved in inducing protective immune responses, with a view to developing a recombinant protein, synthetic peptide or DNA vaccine (Argiro *et at*. 2000; Eberl *et at*. 1999; Capron and Dessaint 1992; Hooker *et at*. 1995; Inal and Bickle 1995; Kagan and Cahill 1968; Kalinna and McManus 1997; McManus *et at.* 1998; Richter *et at*. 1993; Riveau *et at*. 1998; Scot and McManus 2000; Yang *et at.* 2000). At present no vaccine is available, however the encouraging results suggest that development of a vaccine may ultimately be achievable (McManus 1999).

### Diagnosis and diagnostic methods

Diagnosis is central to all aspects of schistosomiasis (reviewed by Bergquist (1992); Feldmeier and Poggensee (1993); Maddison (1987); Maddison (1991); Rabello (1997); Tsang and Wilkins (1997). Decisions on individual and community treatment, estimations on prognosis and assessment of morbidity, evaluation of chemotherapy and control measures are built on the results of diagnostic tests. Until now, there is no ideal gold standard; the ideal test should have a high sensitivity and specificity and give qualitative and quantitative information. It should also be easy to perform, without a need for skilled personnel or expensive equipment, and yield reproducible results, which in turn should be simple to interpret. In the following paragraphs, some direct and immunological methods will be discussed.

### Direct parasitological methods:

The diagnosis of schistosomiasis is usually based on the microscopic detection of eggs in urine or stool. The eggs of *Schistosoma* species are characteristic and easy to identify. Urine filtration and centrifugation or the sedimentation enables the diagnosis of urinary schistosomiasis while stool examination by the Kato-Katz thick smear method enables the diagnosis of intestinal schistosomiasis. Egg excretion shows a considerable day-to-day fluctuation and light infections are easily missed by a single routine examination. Therefore, parasitological examination needs to be repeated several times to get an accurate impression of the individual intensity of infection. Both methods have a high specificity, but lack accuracy and sensitivity.

### Immunological methods:

Immunological methods are widely used to test for the presence of host antibodies directed against parasite life cycle stages such as schistosomula, adult worms and eggs, as well as for the detection of circulating schistosome antigens. There is a wide range of serological assays to detect antibody reactivity (e.g. the use of crude, purified or recombinant antigens and the detection of specific antibody isotypes). However, the currently available serological assays based on the detection of antibodies do not discriminate between active and previous infection or re-infection, intensity of infection and effect of chemotherapy. Antibodies persist in chronic infections and antibody levels do in general not significantly change after chemotherapy.

Antibody profiles are of interest for epidemiological research purposes (Dunne *et al*. 1992; Hagan *et al*. 1991). Serology is more suitable in specific situations like diagnosis of *Schistosoma* infection in incidentally exposed individuals (tourists) or in areas where the prevalence of schistosomiasis is so low that parasitological methods fail (Deelder *et al.* 1989b; Polderman and De Caluwe 1989). Hamilton *et al.* (1998) discussed the potential advantages and disadvantages of specific antibody reactivity in more detail. Antigen detection methods generally involve capture of the antigen in an antibody sandwich, the antibodies being monoclonals or polyclonals with specificity for repeating epitopes on schistosome-specific antigens. All life cycle stages appear to excrete/secrete specific antigens e.g. cercarial/schistosomular antigens, adult worm antigens and egg antigens. Most research has concentrated on circulating anodic antigen (CAA) and circulating cathodic antigen (CCA), which are released into the bloodstream of the host by regular regurgitating of the undigested contents of the parasite gut. The development of monoclonal (MAb)-based sandwich ELISA's has made it possible to detect CAA and CCA in a sensitive and specific manner (De Jonge *et al*. 1990; Deelder *et al.* 1989a; Deelder *et al.* 1994). Large amounts of CAA and CCA are excreted already early during the development of the parasite. The antigens are detectable in human sera 4-5 weeks after infection (Gundersen *et al.* 1992; Van Lieshout *et al.* 1997). It was shown that the concentration of these antigens correlates with the number of living worms (De Jonge 1990) and antigen levels correlate significantly with egg excretion and decrease rapidly following successful treatment (De Jonge *et al*. 1989; Shaker *et al*. 1992). CAA and CCA levels do not reflect the tissue egg load.

Several studies have focused on the detection of egg antigens. Using a MAb-based ELISA, circulating soluble egg antigen (SEA) was demonstrated both in serum and in urine of infected individuals. Various authors have reported significant correlations between the amount of SEA detected and egg output (Nibbeling *et al.* 1998c; Nourel Din *et al.* 1994b; Kahama *et al.* 1999; Nibbeling *et al*. 1998b; Nibbeling *et al.* 1998a). Urine SEA levels of S. *haematobium* infected individuals correlate with egg counts, haematuria and urine tract pathology (Kahama *et al.* 1999). SEA levels are independent of the day-to-day fluctuation in egg excretion (Kahama 1998), however light infections are easily missed.

The parasite adapts to different environments and interacts in a specific way with the host. The parasite expresses and excretes many stage-specific antigens. For most of these antigens it has been found that they are glycoconjugates and that the immunoreactive part is located in the carbohydrate chains (Van Dam and Deelder 1996; Hokke and Deelder 2001). These glycoconjugates play an important role in the evasion mechanisms of schistosomes to withstand the immunological measures of the host.

Schistosomes contain and synthesize a broad array of glycoproteins, glycosphingolipids, polysaccharides and GPI-anchors. Some of these glycoconjugates are excreted by the parasite or presented on the surface of the parasite. The expression of many of these glycan structures is developmentally regulated and stage- specific as shown by the diversity of lectins that bind to the surface of the organism (reviewed by Van Dam and Deelder (1996)). Each of the different life-cycle stages interacts in a specific way with the host, thereby manipulating the physiological/immunological mechanisms of the host for its own benefit.

### N-glycans

Schistosome-derived glycoproteins contain *N*-glycans that show strong similarities with structures found in mammals. Both oligomannosidic-and complex-type *N*-linked glycans are found in adult worms and eggs. The oligomannosidic-type *N*-glycans have a typical Man₅₋₉GlcNAc₂-Asn structure (Nyame *et al*. 1988a) and the complex-type *N*-glycans are di-, tri- or tetra-antennary (Nyame *et al.* 1989). Many of the complex-type *N*-glycans contain several terminal structures, such as Galβl-4GlcNAc (LacdiNAc, LDN) and Galβl-4(Fucαl-3)GlcNAc (LDN-F), which are primarily present on di-antennary chains (Nyame *et al.* 1989). The tri- and tetra-antennary *N*-glycans contain branches containing poly-Galβl-4GlcNAc (LacNAc) chains carrying Galβl-4(Fucal-3)GlcNAc (Lewis^{X}, Le^{X}) epitopes (Levery *et al.* 1992). The terminal structures are summarized in Table 1.

Glycoproteins from *S*. *mansoni* and *S*. *japonicum* eggs contain unusual core modifications such as β2-xylose (β2-Xyl) residues and α3-fucose (α3-Fuc) residues linked to the core (Khoo *et al.* 1997b). These structures are not found on *S*. *japonicum* adults and cercariae. On the other hand, a majority of the *N*-glycans from S. *mansoni* cercariae, but not the adults are core xylosylated (Khoo *et al.* 2001).

*S. japonicum* egg glycoproteins contain core structures carrying β2-Xyl, α3-Fuc and α6-fucose (α6-Fuc) structures so far not described in any other species. The α3-fucosylated and β2-xylosylated *N*-glycans are also found on other helminth-, plant-, insect-, and mollusc glycoproteins, but not on mammalian glycoproteins. In addition, *N*-glycans isolated from eggs contain terminal (HexNAc)₃ stretches substituted with repeating Fuc residues. The *S*. *mansoni N*-glycans have an extended difucosylated oligosaccharide, but the terminal difucosylated GalNAc has not been demonstrated in *N*-glycans from *S. japonicum.* The *N*-glycans isolated from *S*. *mansoni* and *S*. *japonicum* cercariae are both dominated by Lewis^{x} termini. Studies with different lectins have shown that cercariae, miracidia and adults contain glycoproteins that carry complex type glycans carrying one and/or two fucosylated determinants (Mansour 1995; Mansour 1996; Negm 1996)

### O-glycans

Many membrane-associated and secreted glycoproteins derived from adult schistosomes have simple mucin type *O*-glycans that are identical with structures found on mammalian glycoproteins, including GalNAcal-Ser/Thr and Galβl-3GalNAcal-Ser/Thr (Nyame *et al*. 1988b). Newly transformed schistosomula do not express this disaccharide form. In addition, many intracellular glycoproteins from *S*. *mansoni* adults and schistosomula have been shown to contain *O*-linked GlcNAc, which is a common modification found in a large number of mammalian proteins (Nyame *et al*. 1988b).
In addition to the relative simple *O*-glycans, the circulating antigens excreted from the gut of the adult worm contain more complex *O*-glycans. The major glycan part of the circulating cathodic antigen (CCA) consists of an *O*-linked poly-Lewis^{x} carbohydrate chain with approximately 25 repeating units, containing GaINAc as the reducing terminal monosaccharide (Van Dam *et al,* 1994).

The poly-Lewis^{x} chains are attached to the protein backbone predominantly via core 1 and/or core 2 *O*-glycan structures. In addition, a minor carbohydrate fraction consists of disaccharide to hexasaccharide structures having a Galsl-3GalNAc core in common (Van Dam *et al*. 1994).

*S. mansoni* circulating anodic antigen (CAA) is a unique threonine-linked polysaccharide consisting of a repeating motif of polymeric GalNAc residues substituted with GlcA, probably connected to the protein via a, yet unknown, core saccharide with GlcNAc at the reducing end (Bergwerff *et al*. 1994).

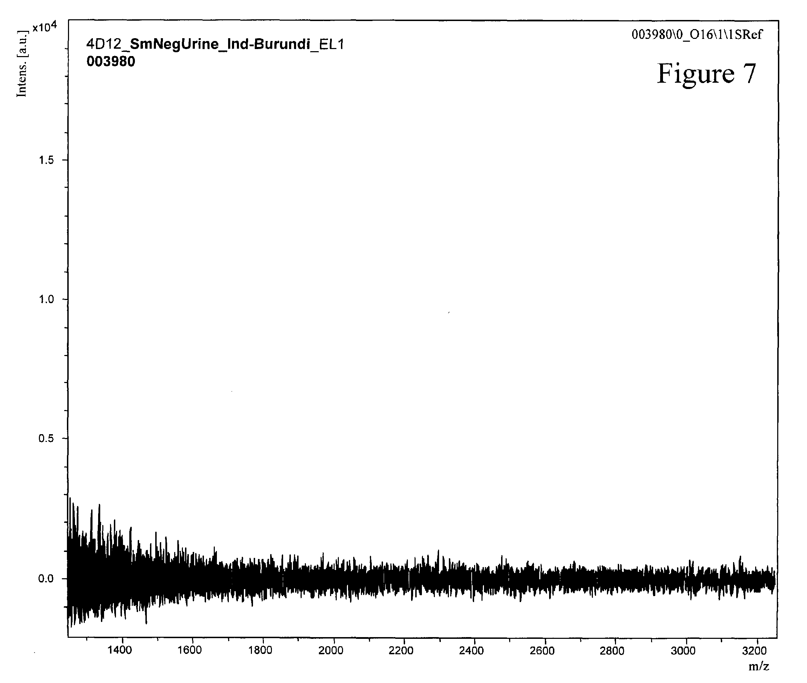

With respect to *O*-glycosylation of cercarial proteins, the *O*-glycans isolated from the glycocalyx have an unusual backbone of a trisaccharide-repeating unit, -3GalNAcβ1-4GlcNAcβl-3Galαl- substituted with di- and trifucosyl residues (Khoo *et al*. 1995). These multimeric glycans are attached via core 1 and/or core 2 (Figure 7) (Khoo *et al*. 1995). Recently, other cercarial *O-*glycans have been identified that terminate with GlcNAc, LacNAc or Lewis^{x} carried by unusual di-antennary core structures with multiple branched Gal residues (Huang *et al*. 2001). Finally, *O*-glycans isolated from *S*. *mansoni* eggs were found to carry multifucosylated HexNAc sequences as the cercarial *O-*glycans.

### Glycolipids

Schistosome glycolipids contain GalNAcβ1-4Glcβ1-Cer as a core structure, the so-called "schisto core", rather than Ga1β1-4Glcβ1-Cer which is present on sphingoglycolipids in higher animals. This disaccharide is an interesting feature of the neutral glycolipids in adult schistosomes. The simple core structure is extensively modified in egg glycolipids from *S*. *mansoni* and *S. japonicum,* where they contain a repeating unit of -4(Fucαl-2Fucαl-3)GlcNAcβ1- and a terminus of ±Fucαl-2Fucαl-3GalNAcβ1.

The terminal difucosylated GalNAc is absent in egg glycolipids of *S*. *japonicum* (Khoo *et al*. 1997a). Recently, complex antigenic glycolipids from the cercarial stage of *S*. *mansoni* have been analysed. They contain large amounts of terminal and 3-substituted galactose (Wuhrer *et al.* 2000), and to a lesser extent glycolipids similar to those found in eggs (Khoo *et al*. 1997a; Wuhrer *et al*. 2000) The Lewis^{x} and pseudo-Lewis^{y} glycolipids are stage-specifically expressed by the cercariae and not by the adult worms or eggs (Wuhrer *et al*. 2000).

### GPI-anchored glycoproteins

*S. mansoni* contain many glycoproteins anchored to membranes via GPI. Glycoproteins containing the GPI anchor are for example alkaline phosphatase and acetylcholinesterase, Sm18, Sm23, Sm25, Sm32, Sm38 and Sm200 (Camacho *et al*. 1996; Jones *et al.* 2002; Koster and Strand 1994; Pearce *et al.* 1991b; Sauma and Strand 1990). Sm18, Sm32 and Sm38 are present in the adult worms, but not in 7-day-old lung stages, indicating that the expression is developmentally regulated. Antibodies of chronic infected mice or of mice vaccinated with irradiated cercariae showed reactivity with these glycoproteins that are released from the adult worms in membrane vesicles. The functional significance of these GPI-anchored proteins is unknown; their release from the surface of the schistosome may contribute to the immune evasion. Sm200 is one of the few antigens that are exposed on the surface of the adult worms following treatment with praziquantel (Hall *et al.* 1995; *Sauma et al.* 1991).

### Proteoglycans

Different types of proteoglycans are found in adult worms of *S*. *mansoni* and *S*. *haematobium* such as heparin/heparan sulphate and chondroitin sulphate. The presence *of* heparin-like polysaccharides is thought to prevent entrapment *of* the parasite by the host's blood clotting process (Hamed *et al.* 1997; Robertson and Cain 1985). Hyaluronic acid is only found in *S. mansoni* adult worms.

### Schistosome glycosyltransferases

Based on the structural data, schistosomes must have many different glycosyltransferases. Relatively few of these enzymes or their activities have so far been described. Some of these are described herein below. A β4-GalT which forms LacNAc sequences and a B4-GaINAcT which generates LDN sequences have been identified in *S*. *mansoni* adult worms (Rivera-Marrero and Cummings 1990; Srivatsan *et al*. 1994). The β4-GalNAcT is capable of utilizing the same acceptor *in vitro* as the schistosomal B4-GaIT. Therefore, the relative activities of these two enzymes may regulate the LacNAc versus LacdiNAc pathway (Van den Eijnden *et al.* 1997). A similar type of β4-GalNAcT is present in cercariae, miracidia and mother sporocysts of the avian schistosome *Trichobilharzia ocellata* and in its snail host *Lymnea stagnalis* (Neeleman *et al.* 1994).

Of particular interest are the FucT's since fucosylation is a common structural theme for most schistosomal glycoconjugates. A major structural feature of schistosomal glycoconjugates is the Fucal-3GlcNAc linkage. In the complex-type *N*-glycans of membrane glycoproteins and *O*-glycans of CCA from adult schistosomes, this fucosyl linkage is present in the Lewis^{x} antigen or in its analogue variant LDN-F. (Trottein *et al.* 2000) reported the cloning of an a3-FucT from *S*. *mansoni*. Besides the identified α3-FucT, other unidentified a3-FucT's must be present to synthesize structures like Fucal-3GalNAc and Fucαl-3Galβ1-4GlcNAc both present in schistosomes (Khoo *et al*. 1995; Khoo *et al.* 1997a; Wuhrer *et al.* 2000).

In addition, an α2-FucT that can synthesize Fucal-2Fuc sequences has been found in cercariae of the schistosome *T. ocellata* (Hokke *et al.* 1998). On the basis of the occurrences of the multifucosylated structures on various schistosomal glycoconjugates, one or more similar α*2*- FucTs should be present in *S*. *mansoni.* This enzyme can be distinguished from the human α*2*- FucTs which are involved in the synthesis of the blood group H-antigen (Fucal-2Galβ1- 4GlcNAc) on red blood cells (Henle *et al*. 1990), or from a novel α2-FucT from *C. elegans*, that preferentially acts on Galβ1-4Xyl (Zheng *et al*. 2002).

The expression of glycosyltransferases is probably developmentally regulated, since several glycan structures are unique for different stages of the parasite (e.g. no expression of the β4- GalT activity in cercariae in contrast to B4-GalT in adults). The identification and the expression of the different glycosyltransferases in schistosomes provide information about their fundamental role in the parasite development and host-pathogenesis.

Furthermore, the different enzymatic properties of several extracts derived from mammals, invertebrates and helminths can be applied to synthesize terminal carbohydrate structures, which are a part of the glycoconjugates that are excreted or expressed by the parasite.

### Function of glycoconjugates in schistosomes

Schistosomal glycoconjugates, either excreted or expressed on the surface, are key modulators or targets of the host immune system. The expression of many of these glycans is developmentally regulated and stage-specific, but their fundamental role in parasite development and host pathogenesis is not clear. Probably, the glycoconjugates mediate specific parasite defence or survival mechanisms (in the form of immunomodulation/suppression, host mimicry (glycomimesis)) or perhaps involvement in host lectin binding, targeting and signalling. Any role in disease manifestations may arguably be incidental, due to improper or excessive host responses to the offensive antigens. It is also likely that the different *Schistosoma* species differ in several ways in terms of their glycoconjugate structures.

### Structural and modulatory roles of glycans

The unique complex oligosaccharide structures isolated from the glycocalyx, which covers the entire surface of the cercariae may play a role in the mechanical stabilization of the surface of the schistosome larvae as they progress from the snail intermediate host, through fresh water, into their definitive host (Nanduri *et al.* 1991). Likewise, the glycocalyx of miracidia which is found to be quite similar to the glycocalyx of cercariae may have the same function (Abdul- Salam and Mansour 2000; Chiang and Caulfield 1988).

In addition, the glycocalyx is involved in complement activation by the alternative pathway and stimulates the serological response after immunization. It remains to be investigated whether the multi-fucosylated structures isolated from the glycocalyx *of* cercariae contribute to the known Thl down-regulation in schistosomiasis, through a mechanism similar to that described for Lewis^{x} (Thomas and Harn 2004). Recently, it was shown that some neoglycoconjugates carrying a multi-fucosylated structure have the capacity to stimulate monocytes to release cytokines, and in particular IL-10, IL-6 and TNF-α (Van der Kleij *et al.* 2002). A proposed model for immunomodulatory functions *of* parasite sugars is transcriptional regulation *of* cytokine expression by ligand non- specific entry into cells.

The membrane *of* adult worms is highly regenerative, even after complement attack. Despite the intense humoral response to carbohydrate antigens, this immune attack ineffective in destroying the mature worms. This is unusual, since antibodies to schistosomal carbohydrate structures present in sera of patients are capable of fixing complement. Although these antibodies may not be effective in destroying the tegumental membrane of the adult schistosome, they may nevertheless be critical to the parasite's sensitivity to complement-induced lysis in the presence of the drug praziquantel. Finally, CAA derived from the gut *of* the adult schistosome can bind to Clq stalks with characteristics similar to the first complement component Clq and Clq receptor interaction, which may possibly block binding *of* Clq to the Clq receptor and may protect the gut against complement-mediated attack (Van Dam *et al.* 1993b).

### Glycans in recognition and adhesion

As described above, schistosomes contain a high variety of glycoconjugates. Some of these glycoconjugates contain glycans that are identical to glycans found in the host, while others are unique for the parasite. Probably, some *of* these glycoconjugates will be recognized by the innate immune system as foreign antigens, others as host molecules and therefore both can interact with host lectins.

Adhesion molecules have been implicated in several events in *Schistosoma*-host interactions, including granuloma formation (Jacobs *et al.* 1998). Increased serum concentrations of soluble intercellular adhesion molecule-1 (sICAM) and of soluble E-, but not of soluble P- and L- selectins were detected in S. *mansoni* infected individuals and these levels correlated with the disease severity. Besides, the levels *of* sICAM remained elevated for months after treatment with praziquantel (Esterre *et al*. 1998; Secor *et al*. 1994).

Adhesion molecules belonging to the selectin family participate in antibody-dependent cell mediated cytotoxicity mediated by human eosinophils towards schistosomula of *S*. *mansoni* (Nutten *et al.* 1999). Host-soluble L-selectin binds to target ligands on the surface membranes *of* the miracidia, thereby possibly impeding the release *of* soluble antigens from the egg and down regulating the granulomatous pathology. The adhesion *of* the egg to activated human umbilical vein endothelial cells, which is E-selectin mediated could be inhibited by an anti-Lewis^{x} MAb (Lejoly-Boisseau *et al*. 1999). Microvascular endothelial cells express specific adhesion molecules on their surface including E-selectin and vascular cell adhesion molecule (VCAM-1). These molecules interact with counter-receptors on circulating immune cells, thus permitting the binding and the transmigration of leukocytes at sites *of* inflammation. It is suggested that schistosomulum- endothelium interactions may have important immunological consequences in vivo by down- regulating the expression of E-selectin and VCAM-1. Schistosomula may use this to escape the immune system by controlling leukocyte recruitment to the lungs (Trottein *et al.* 1999).

Mannan-binding lectin (MBL), a serum protein that is part *of* the innate immune system, binds to surface carbohydrates of cercariae and adult worms (Klabunde *et al.* 2000). These authors showed that in *vitro* MBL, in association with the serine proteases MASP-1 and MASP-2, is capable of fixing complement on the schistosomal tegument and *of* activating the complement cascade via the MBL pathway. More recently it was found that the collectin SP-D binds to the surface of schistosomula via fucosylated glycoconjugates (Van de Wetering *et al*. 2004) and that GalNAcβ1-4GlcNAc (LDN) on schistosome eggs serves a pathogen-associated molecular pattern for immune recognition by galectin-3 (Van den Berg *et al.* 2004).

Another way around whereby lectins present in the parasite interact with carbohydrate structures present on glycoconjugates *of* mammalians can also play a role in parasite-host interaction. However, only limited knowledge is available on trematode lectins, therefore the functions *of* these lectins have not been elucidated, yet. So far, no C-type lectins have been found in schistosomes. It is known that neoglycoproteins bind to the surface or to proteins secreted by most stages *of* the schistosome life cycle, indicating that lectins must be present. A MAb directed against human E-selectin was found to bind to the surface of *S*. *marcsoni* schistosomula and this binding could be inhibited by E-selectin. It is proposed that the schistosome can adsorb host-molecules (antibodies and complement components) and MHC class I antigens to avoid immune recognition (Trottein *et al*. 1999).

Lectin activity has been found on the surface and in the acetubular glands of cercariae of the bird schistosome, *Trichobilharzia szidati* (Horak *et al*. 1997). The authors speculated on a role of this lectin in recognition of connective tissue components or in molecular interactions of the parasite with the immune system of the host.

Glycoproteins and glycolipids of schistosomes contain many different types of complex glycans. The structures of many of these glycans have been determined by chemical approaches from glycoprotein and glycolipid preparations of the main schistosome developmental stages: cercariae, adult worms and eggs (see literature, review Hokke and Deelder). To a number of the terminal di-, tri- and tetrasaccharide elements mentioned in Table 1, we have generated monoclonal antibodies from a large library created from schistosome-infected mice. Chemically and enzymatically synthesized carbohydrate elements were used to screen this library. mAbs were classified as specifically binding to e.g. LDN, LeX, LDN-F, F-LDN-F etc. A particular mAb (114-4D12) that has previously been selected for use in a sandwich ELISA assay for schistosome antigen detection (together with mAb 114-5B1) binds to the synthetic Fucα1-2Fucα1-3GlcNAc element.

To determine in more detail to which (secreted) antigens from schistosome eggs 114-4D12 exactly binds, a glycoprotein family has been affinity purified from S. *mansoni* soluble egg proteins with this antibody. The O-glycans of this protein preparation were released, and their structures were investigated by various MS-based techniques. To determine which exact structures in the heterogeneous pool contained the epitope for 114-4D12, the released glycans were re-applied to the affinity column. The combined structural data indicate that the structures of the specifically bound glycans are Fucα1-2Fucα1-3GalNAcβ1[-4(Fucα1-2Fucα1-3GlcNAcβ1-)]_{1,2}Hex. From a second approach, using direct affinity selection of O-glycans released from egg glycoproteins by reductive β-elimination, a larger series of glycans containing in addition extensions of the structures found with the first approach was found to bind to the mAb.

In the present invention it has been shown that pathogens and in particular the various developmental stages of helminths produce unconjugated oligosaccharides. These pathogen specific unconjugated oligosaccharides can be detected directly in a sample. However, typically some type of pre-enrichment and/or purification is performed prior to the analysis. In a preferred embodiment the sample is incubated with a binding molecule that specifically binds to carbohydrate structure present on an oligosaccharide. The unbound fraction is at least partly washed away whereupon the bound oligosaccharide is released and unconjugated oligosaccharide is analysed. It is preferred that the binding molecule has a high affinity for unconjugated oligosaccharide. In a preferred embodiment the binding molecule is specific for a pathogen specific structure. Preferably, the binding molecule comprises a specificity for a structure as depicted in figure 9, figure 10 or table 1. Preferably the binding molecule comprises a specificity for a structure as depicted in table 1 or a functional art, derivative and/or analogue thereof. Many different types of molecules are able to specifically bind oligosaccharides. Such molecules for instance comprise receptors and/or ligands that are specific for one or more oligosaccharide structures such as plant and animal lectins. Other binding molecules comprise (modified) enzymes that catalyse the synthesis or breakdown of particular oligosaccharides and biotechnologically designed and produced synthetic binding molecules (such as iMabs). A preferred type of binding molecule is an antibody or a functional part, derivative and/or analogue thereof. There are numerous antibodies that can specifically bind a structure on an oligosaccharide. Preferred examples of (pathogen specific) antibodies are:

| *Antibody* | *Specificity* [reference] |
|---|---|
| 114-4D12 | Fucα1-2Fucα1-3HexNAc |
| | [Nourel Din et al (1994) Am J Trop Med Hyg 50:585-94] |
| 114-5B1 | LDN-DF |
| | [Van Remoortere et al (2000) Glycobiology 10:601-9; Robijn et al (2005) Parasitology 130:67-77] |
| 290-4A8 | LDN-DF |
| | [Van Remoortere et al (2000) Glycobiology 10:601-9; Robijn et al (2005) Parasitology 130:67-77] |
| 259-2A1 | LDN |
| | [Van Remoortere et al (2000) Glycobiology 10:601-9] |
| 273-3F2 | LDN |
| | [Van Remoortere et al (2000) Glycobiology 10:601-9] |
| 291-2G3 | LeX |
| | [Van Remoortere et al (2000) Glycobiology 10:601-9; Van Roon et al (2005) Thesis Leiden Univ, Chapter 3] |
| 291-2F3 | dimerLeX |
| | [Van Roon et al (2005) Thesis Leiden Univ, Chapter 3] |
| 54-5C10 | trimerLeX |
| | [Deelder et al (1996) Parasitology 112:21-35; Van Roon et al (2005) Thesis Leiden Univ, Chapter 3] |
| 114-4C1 | GlcNAc |
| | [Van Roon et al (2005) Thesis Leiden Univ, Chapter 7] |
| 128-1E7-C | F-LDN-F |
| | [Robijn et al (2005) Parasitology 130:67-77, Van Roon et al (2005) Thesis Leiden Univ, Chapter 7] |
| 128-3G12-A | Fucα1-3GlcNAc |
| | [Van Roon et al (2005) Thesis Leiden Univ, Chapter 7] |
| 291-5D5-A | F-LDN |
| | [Robijn et al (2005) Parasitology 130:67-77] |
| 290-2E6-A | LDN-F |
| | [Van Remoortere et al (2000) Glycobiology 10:601-9; Robijn et al (2005) Parasitology 130:67-77] |
| 100-4G11-A | Man₃GlcNAc₂ |
| | [Van Remoortere et al (2003) Glycobiology 13:217-225] |
| 25-3D 10-A | GlcAβ1-3GalNAc |
| | [Vermeer et al (2003) Parasitol Res 90:330-6] |
| M2D3H | Fucal-3GalNAc |
| | [Kantelhardt et al (2002) Biochem J 366:217-23] |
| SMLDN1.1 | LDN |
| | [Nyame et al (2000) Exp Parasitol 96:202] |
| SMLDNF1 | LDN-F |
| | [Nyame et al (1999) Glycobiology 9:1029] |
| Sm 10 27 12 mAb | LeX |
| | [Lejoly-Boisseau et al (1999) Exp Parasitol 91:20-9] |
| E.5 | LeX |
| | [Ko et al (1990) Proc Natl Acad Sci USA 83:4159-63] |
| BR96 | LeY |
| | [Jeffrey (1995) Nat Struct Biol 2:466-71] |

In the present invention it was found that the presence of pathogen related unconjugated oligosaccharide in a body fluid of an animal is an indication that the animal is infected by a pathogen related disease. More in particular, it was found that the pattern of unconjugated oligosaccharide in said body fluid changes with the health (disease) status of the animal. Thus by analysing an oligosaccharide pattern in said body fluid, or by and/or typing unconjugated oligosaccharide therein, it is possible to determine whether the animal does or does not have disease or does or does not have a subclinical infection. By comparing said pattern or said typing with a reference it is further possible to diagnose pathogen related disease or said infection.

It is further possible to quantitate differences between patterns. For instance, a pattern of a test sample can be compared with a pattern of a reference of a healthy animal. Differences between the patterns can be quantitated in various ways and be used to determine the severity or stage of the disease. This can be coupled for instance to prognosis of the disease. Differences in patterns can be quantitated by counting the number of different unconjugated oligosaccharides in the patterns. Quantitation can also be done by comparing relative or actual amounts of one or more types of unconjugated oligosaccharides, this quantitation can be done in the comparison of patterns but also when specific types of unconjugated oligosaccharides are analysed. Analysis of patterns may include conjugated oligosaccharide as long as said analysis of said pattern comprises the use of at least one unconjugated oligosaccharide. The body fluid is preferably urine. Urine is easily obtained in large quantities and is a reservoir wherein unconjugated oligosaccharides accumulate. The disease is preferably a helminth infection. In this case the presence of the helminth (species-) specific unconjugated oligosaccharides is a marker of infection. In the case of schistosomiasis the quantitation of egg-specific unconjugated oligosaccharides can be used as a marker for morbidity, for instance by relating the amount of unconjugated oligosaccharides to the amount of eggs present in the host. In schistosomiasis, the inflammatory granulomas formed around tissue-trapped eggs form the basis of the main pathology. Samples derived from schistosome infected animals comprise as mentioned above, CAA. Binding molecules specific for CAA typically recognize a structure present on a saccharide chain of said CAA. In one embodiment a sample suspected of containing pathogen specific unconjugated oligosaccharide is incubated with such a binding molecule for CAA, whereupon bound oligosaccharide is analysed for the presence of pathogen specific unconjugated oligosaccharide. In yet another embodiment, a method for the detection of unconjugated oligosaccharide is used for the detection of a pathogen in an animal. Thus in one embodiment the invention provides the use of unconjugated pathogen specific oligosaccharide as a marker for pathogen related disease. The pathogen is preferably a helminth. The helminth is preferably a schistosome.

No animal is immune to infection by pathogens. A sample of the present invention can therefore be derived from any type of animal. In a preferred embodiment an animal is a farm-animal or a pet. In another preferred embodiment, said animal is a human.

### Examples

### Detection of schistosome oligosaccharides in infection urine

Glycoconjugates of schistosomes contain a large variety of different types of complex glycans. The structures of many glycoprotein and glycolipid derived glycans have been determined in the past decade, mainly by mass spectrometry-based approaches, of the main schistosome developmental stages: cercariae, adult worms and eggs (review Hokke and Deelder, and references therein).
These complex glycoconjugate glycans contain elements that have been implicated in various immunological processes involved in schistosomiasis. These elements are immunogenic to the (human) host by inducing humoral as well as cellular immune responses. Therefore, schistosome glycoconjugates are of considerable interest as diagnostic and therapeutic targets. Some glycan elements produced by schistosomes, either stage specifically or not, seem to be unique for the schistosome or related species, whereas other are occurring more widespread in nature, or may even be shared with the human host. The more unique structural elements (see Table 1) include the multi-fucosylated Fucα1-2Fucα1-3GlcNAc containing structures and CAA, whereas LDN, LDN-F and Le^{x} are less unique and occur also on mammalian glycoproteins. In the present invention, however, the latter structures are also present on pathogen specific unconjugated oligosaccharide, since typically the quantity of unconjugated oligosaccharides carrying such structures is increased, when compared to sample of animals or plants that is free of said pathogen.
These structures and structural elements are all part of glycoproteins and glycolipids. It is only partly known where exactly in the organism they are expressed and whether they are secreted or not. Hardly any data is available so far about the identity of the underlying proteins. To the terminal glycan elements mentioned in Table 1, we have selected monoclonal antibodies (mAbs) from a large library of mAbs created from schistosome-infected and/or -immunised mice. Chemically and enzymatically synthesized carbohydrate elements were used to screen this library using surface plasmon resonance spectroscopy (BIAcore) and mAbs were classified as specifically binding to e.g. LDN, LeX, LDN-F, F-LDN-F etc.
A particular mAb 114-4D12 has previously been used in a sandwich ELISA assay, together with mAb 114-5B1, for schistosome antigen detection in urine/serum of infected individuals. In IFA studies on frozen sections of schistosome eggs trapped in hamster livers, it was observed that 114-4D12 binds to antigens present in the egg, and in the tissue surrounding the egg. The latter observation indicates that this mAb recognizes secreted egg glycoproteins.

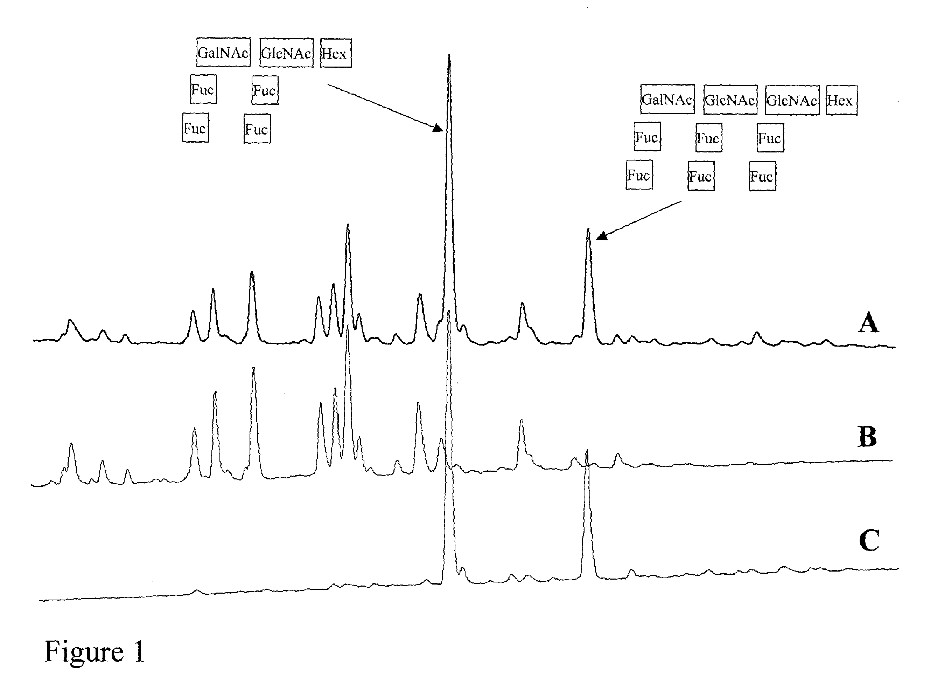

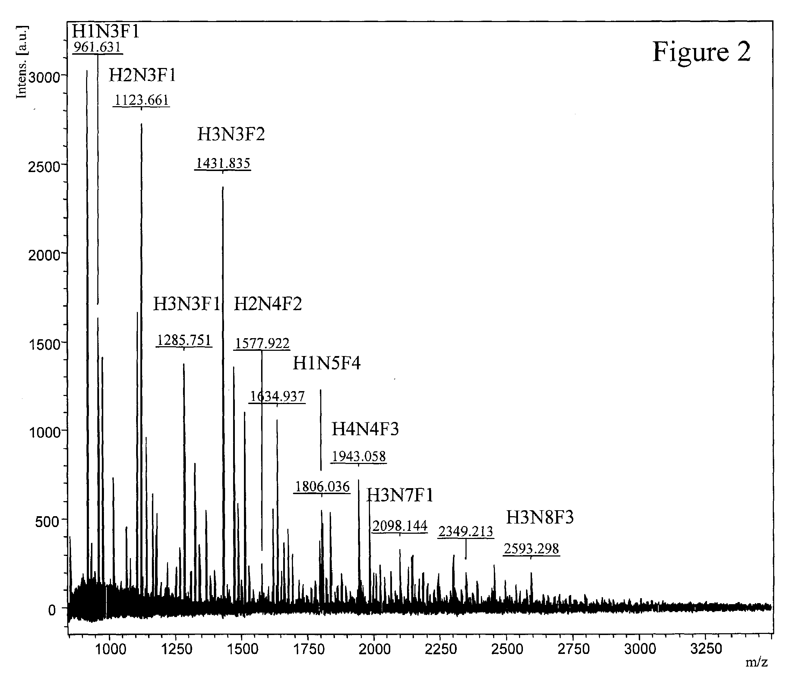

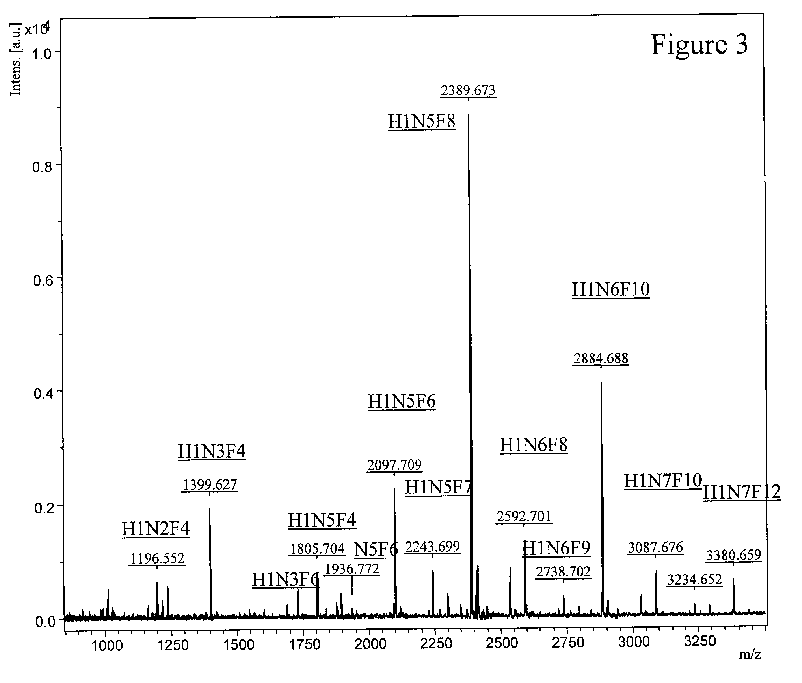

To determine in more detail to which antigens from schistosome eggs 114-4D12 exactly binds, a glycoprotein family was affinity-purified from S. *mansoni* soluble egg proteins (SEA) using 114-4D12 coupled to a Sepharose matrix. The O-glycans of this targeted protein preparation were released by hydrazinolysis and fluorescently labelled, and their structures were investigated by various (LC-)MS-based techniques. The heterogeneous pool of released O-glycans was re-applied to the 114-4D12 affinity column to determine which of the released glycans contained the epitope for this mAb (Fig 1). The combined structural data of MALDI-TOF MS, LC-MS/MS and GC-MS analyses of the different fractions indicate that the structures of the specifically bound glycans are Fucα1-2Fucα1-3GalNAcβ1[-4(Fucα1-2Fucα1-3GlcNAcβ1-)]_{1,2}Hex. In a second approach, using direct affinity purification of O-glycans released from total SEA by reductive β-elimination, a more extensive series of glycans was found to bind to the mAb (Figs 2,3), with masses that indicate similar highly fucosylated structures as in the first sample. Using a series of synthetic oligosaccharides to study by SPR the specificity of the mAb, we observed that 114-4D12 binds to Fucα1-2Fucα1-3GlcNAcβ1-R, but not to Fucα1-3GlcNAcβ1-R, GalNAcβ1-4(Fucα1-2Fucα1-3)GlcNAcβ1- and many other structurally less related molecules. This indicates the terminal Fucal-2Fuca1-3 element is an important factor in the specificity/affinity of the antibody. So far, this element has been identified only in schistosome glycoconjugates.

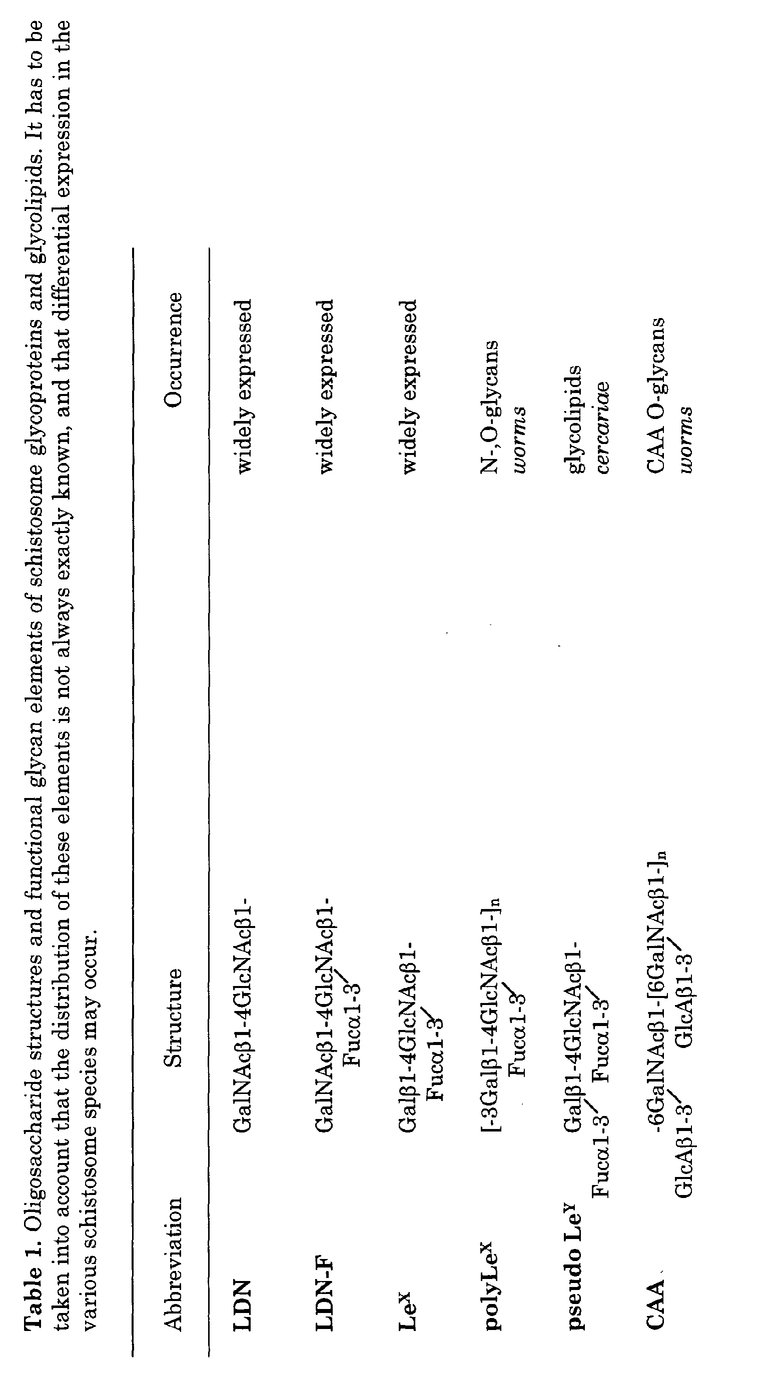

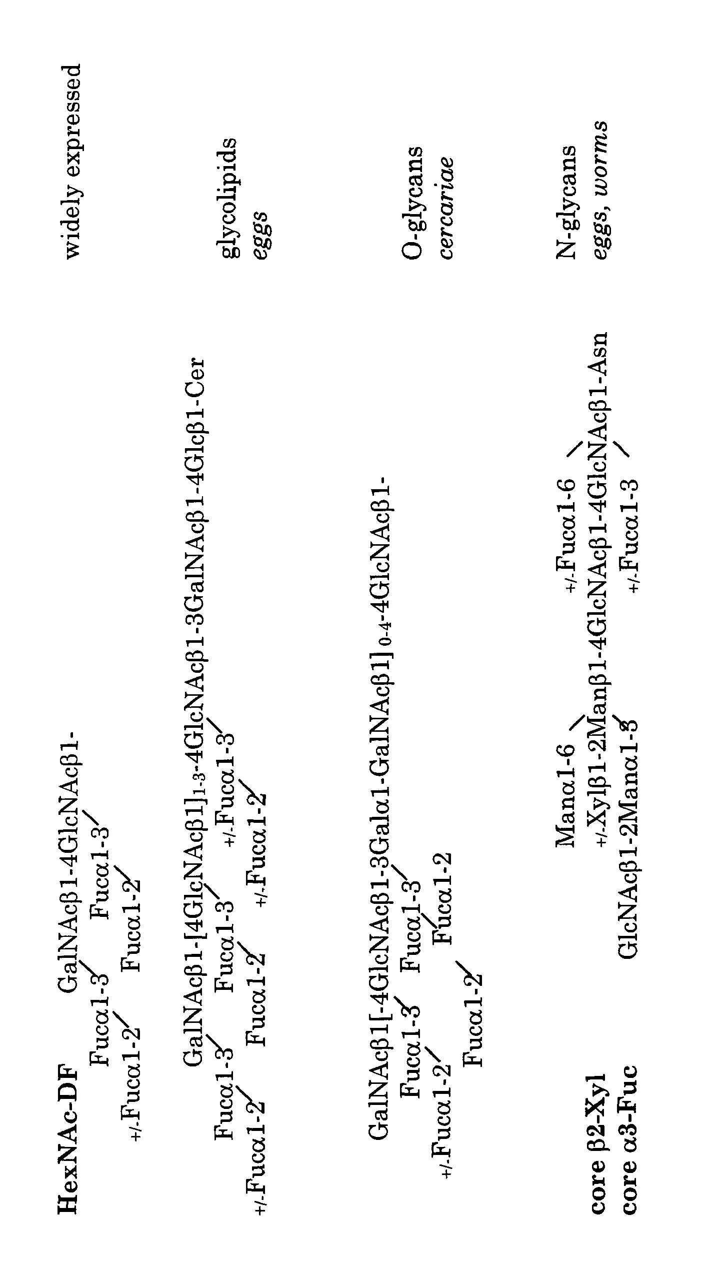

### The following describes the detection of schistosomal free oligosaccharides in schistosomiasis infection urine, and the discovery that these and other free oligosaccharides are produced by S. mansoni eggs.

### Urines

*Schistosomiasis mansoni* and *haematobium* infection urines were obtained from Senegal (pooled samples), Egypt (individual samples), and Burundi (negative controls) and the infection intensity of each individual urine was characterized by conventional methods (number of eggs per gram faeces, epg). The epg value was between 70 (lowest) and 740 (highest) (see Table 2). Pooled urines were filtrated to remove insoluble material, buffered with phosphate buffered saline (PBS) pH 7. Individual urines were not filtered or adjusted, except for addition of 150mM NaCI.

**Table 2. Urine samples from Schistosoma mansoni and/or haematobium infected humans**

| Sample number) (indicated in Fig.6/7 | infection intensity (epg) | urinary CCA level (titre) |
|---|---|---|
| 3977 (pooled samples) | n.d. | n.d. |
| 3980 (neg. control) | 0 | 0 |
| 3981 | 463 | 2048 |
| 3982 | 717 | 1024 |
| 3983 | 70 | 256 |
| 3984 | 153 | 256 |
| 3985 | 740 | 1024 |
| 3986 | 163 | 256 |
| 3987 | 387 | 512 |
| 3988 | 160 | 128 |

### Immuno affinity capturing of urinary compounds

Mab 114-4D12 (IgG1) was covalently coupled to protG-Sepharose beads using pymelimidate according to the protocol described in Sisson and Castor. The coupling yielded 22mg 114-4D12 / ml wet beads. For immunoaffinity capturing of oligosaccharides from pooled urine, 200 µl beads per experiment were used, for individual urines this was 42 µl beads. Prior to use, beads were sequentially washed with 2x10 CV (CV, column volume, 42 or 200 µl), 2x10 CV EL (EL, elution buffer, depending on the experiment 0.04 - 2% formic acid) and 2x10 CV PBS. Beads were incubated overnight (o/n) at 4°C with urine (0.5 - 25 ml), of individual or pooled samples of S. *mansoni*-infected and control individuals. After incubation, beads were washed with 3x10 CV PBS and bound urinary compounds were eluted with 3x10 CV EL. Eluates were neutralised with ammoniumhydroxide and subsequently lyophilised. The dry material was reconstituted in 10 µl water, and of samples of 1 µl, MALDI-TOF mass spectra were recorded using an ATT matrix.

### Mass spectrometric characterisation of eluted compounds

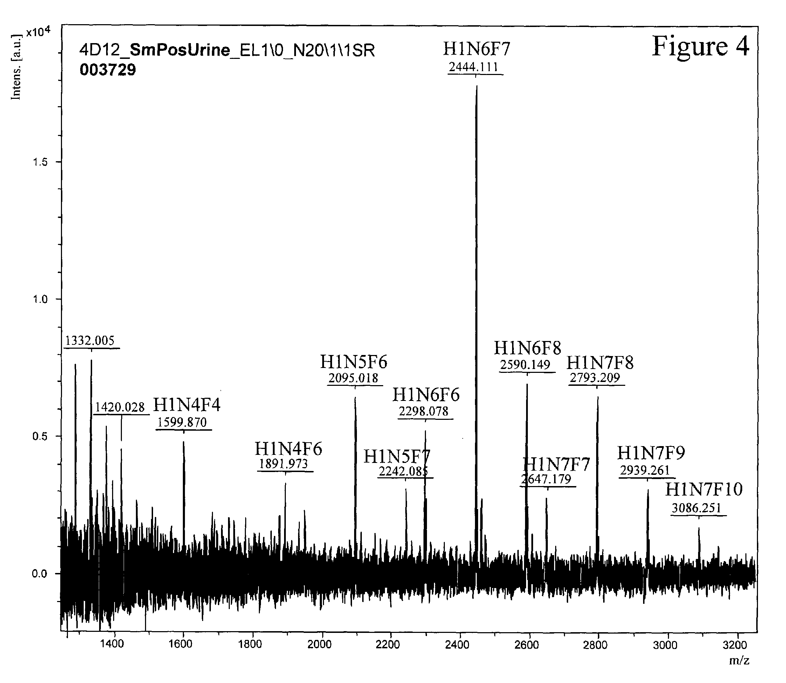

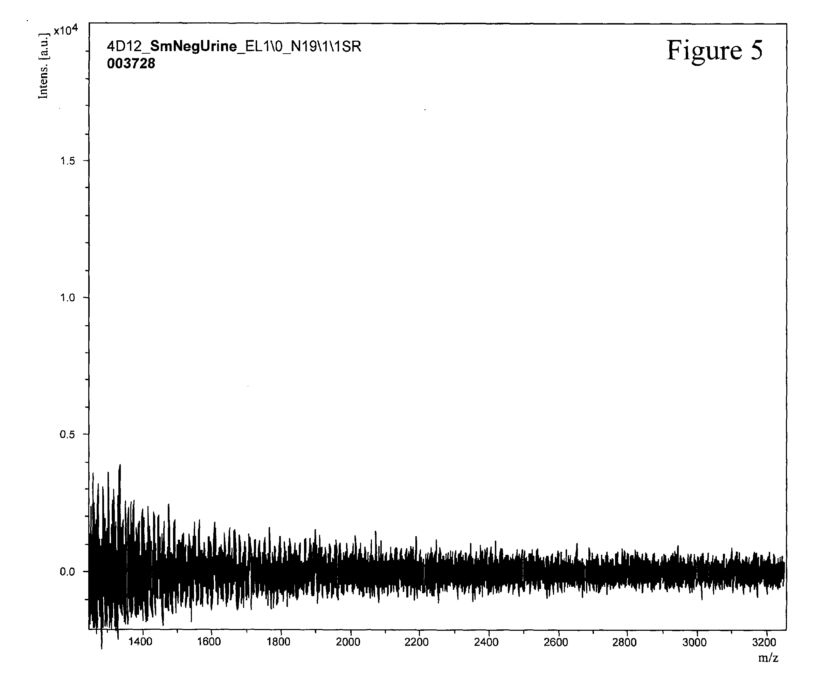

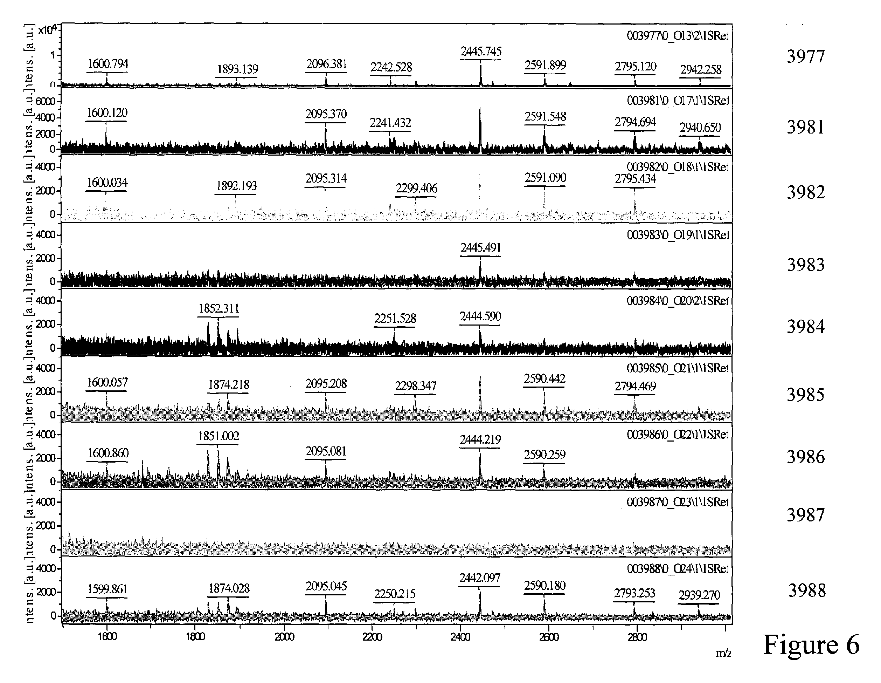

The MALDI-TOF MS of the eluate of the pool of positive urines indicates the presence of a series of highly fucosylated oligosaccharides (Fig.4). The putative compositions of the oligosaccharides in terms of hexose (H), *N*-acetyl-hexosamine (N) and deoxy-hexose (F) were derived from the molecular masses of each compound present. The major signal is derived from (a) compound(s) with the general formula H1N6F7, and the peak pattern indicates also the presence of larger structures with an increased number of N and/or F residues, and the presence of smaller structures with a decreased number of N and/or F residues. These structures appear to form a series of oligosaccharides with similar structural characteristics. The interpretation of the spectra is further supported by the specificity of 114-4D12 (see introduction) and the structures of schistosome egg protein and lipid associated glycans (Table 1, Fig 1), which are in line with the interpretation of the MALDI-TOF MS. The mass spectrum indicated that the affinity purified egg products are free reducing oligosaccharides, and not glycoconjugates, and the masses of all the compounds measured match with the general formula HₓN_{y}F_{z}. This series of fuco-oligosaccharide is related, but not identical, to the series of O-glycans released from egg glycoproteins we found recently. For each of the oligosaccharides present, the deduced structural formula is in line with the presence of the Fucα1-2Fucα1-3GlcNAc epitope of 114-4D12. The MS spectrum of the eluate of a pool of negative control urines does not show any significant signals (Fig 5). Next, elution fractions of individual urines were measured by MALDI-TOF MS (Fig 6). The main signal in each of the MS spectra of the individual urines was from the H1N6F7 structure, similar as in the pooled urine. Also the other signals are mostly present, but sometimes less well detectable because in this case only 1 ml urine per experiment was used, and the infection intensities varied per sample. The MS signals were detected in urine of infected individuals (9/10), but not in urine of non-infected endemic controls (0/10) (Fig. 7).

### Detection of oligosaccharides from incubated eggs

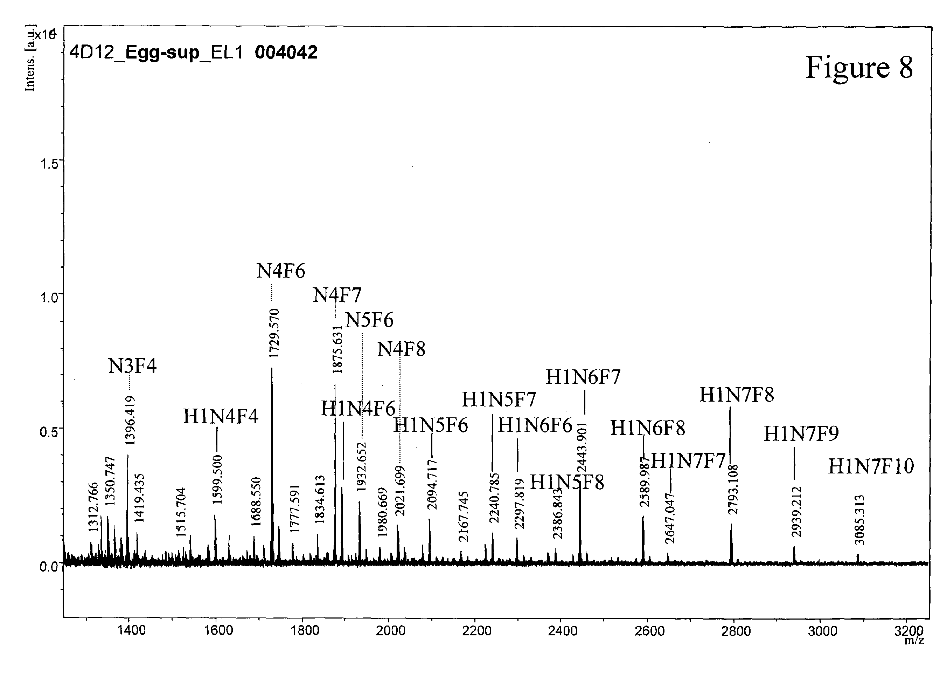

To determine whether the free oligosaccharides are the product of host's metabolic action on e.g. secreted schistosome glycoproteins, or if these oligosaccharides are produced as such by the schistosome, S. *mansoni* eggs and adult worms were isolated from *S. mansoni* infected hamsters. Worms (50 male/female pairs) were incubated 6 h in 4 ml RPMI. Eggs (50.000) were incubated for 4 h in 4 ml RPMI + 1.7% NaCl to minimise hatching. Then, media were collected and incubated overnight with 114-4D12 ProtG-Sepharose beads (85 µl). The eluates (0.04% formic acid) were dried, resuspended in 10 µl water, and 0.5 µl was measured with ATT matrix by MALDI-TOF MS (Fig. 8). The spectrum revealed that the eggs produce the same series of oligosaccharides (as indicated by the mass profile) as found in the positive urines, with H1N6F7 being the major species detected. In addition, a series of oligosaccharides with the formula N3-5F6-8 is present in the egg eluate. These formulas indicate again the presence of Fucα1-2Fucα1-3GlcNAc units, to which 114-4D12 binds, but they lack the hexose residue. This series of oligosaccharides was not detected in infection urine. In the 114-4D12 eluate of the worm incubate no oligosaccharides were detected.

### Diagnosis of schistosomiasis: MS-based oligosaccharide detection as a sensitive alternative to existing methods.

Demonstration of active schistosome infections is either based on demonstration of eggs of the parasite in the faeces or urine of the host (depending on the schistosome species) or on measurement of circulating schistosome antigens. Antibody assays may show a high sensitivity but are notoriously unreliable for demonstration of active infection, as they tend to remain positive long after successful chemotherapy. The ultimate goal is development of a diagnostic assay which has a high sensitivity and specificity and which also detects very low worm burdens. The gold standard against which all assays are (necessarily) measured is - in the case of *Schistosoma mansoni* and S. *japonicum* infection - the quantitative determination of eggs in the faeces using microscopy. In endemic settings in the tropics, particularly in epidemiological studies, a three-fold repeated duplicate Kato-Katz examination of 25 mg faeces samples results in a lower detection level of 7 eggs per gram faeces. In the Netherlands, for imported cases, a glycerol sedimentation technique on 5 gram faeces samples is used, which results (allowing for a certain loss of eggs during the procedure) in a detection of approximately 1 egg per gram faeces. The best circulating antigen assay available so far - the circulating anodic antigen (CAA) ELISA developed in our group - has a lower detection level which corresponds with that of the above-mentioned Kato-Katz procedure.

By definition, the "lowest reproductive parasite load" possible is one worm pair per host; skewed male-female populations may constitute higher worm burdens but will not produce more eggs. There is a general consensus that one *S. mansoni* worm pair produces approximately 300 eggs per day, half of which will be excreted with the faeces. Assuming a faeces production of 500 gram per day, this then results in an egg output of 150:500 = 0.33 egg per gram (or 1 egg per 3 grams faeces). Thus, apart from concentrating and examining larger faeces volumes, there are no diagnostic techniques available which allow reliable detection of minimal worm burdens. Moreover it should be emphasized that both techniques mentioned above are very time-consuming and that egg output strongly fluctuates, necessitating repeated faeces examination on consecutive days in light infections.

In the present invention infected individuals excreting approximately 100 eggs per gram faeces still gave a clearly positive signal. This was based on capturing the diagnostic analytes from 0.5 ml urine, and spotting of 1/10 of the eluate onto a MALDI-target plate. We have shown before that circulating schistosome antigen may be captured with beads from relatively large volumes of urine, resulting in significant improvement of the lower detection level. Passage of 50 ml of urine over a small affinity column and subsequent spotting of the complete eluate would result in a detection level which would be improved one thousand-fold, i.e. which would allow detection of 0.1 egg per gram faeces, already below the amount of eggs produced by one worm pair. Furthermore, the assay-signal could be strongly enhanced by fluorescent labelling of the binding components and then measuring these by fluorescence detection after fractionation with a LC- or CE-system, after having validated the retention time of each peak with mass spectrometry. This would probably lead to a further improvement of approximately 10- to 100-fold of the assay's lower detection level. Thus, even allowing for certain "losses" in improving the assay, it would for the first time seem to be entirely feasible to develop an assay which allows detection even of one worm pair per host.

### Figures

Figure 1 shows (A) normal-phase LC chromatogram with fluorescent detection of the AB-labelled released O-glycans of 114-4D12 captured S. *mansoni* egg glycoproteins. (B) Flow through of these O-glycans after re-application to the same affinity column (C) Eluate of this column.
Figure 2 shows a MALDI-TOF MS (selected mass range) of the flow-through fraction of a 114-4D12 affinity column loaded with the total mixture of O-glycans of *S*. *mansoni* SEA released by β-elimination in the presence of NaBD₄. This procedure yields oligosaccharide alditols (reduced oligosaccharides) with one hydrogen atom replaced by a deuterium atom.
Figure 3 shows a MALDI-TOF MS (selected mass range) of the eluted fraction of a 114-4D12 affinity column loaded with the total mixture of O-glycans of S. *mansoni* SEA released by β-elimination in the presence of NaBD₄. This spectrum indicates which released O-glycans derived from total SEA contain the epitopes for 114-4D12. The formulas of the observed species indicate the presence of extensions of the H1N3F4 compound derived from 114-4D12 affinity-purified SEA. This compound was shown to contain the epitope for 114-4D12 and has the following structure: Fucα1-2Fucα1-3GalNAcβ1-4(Fucα1-2Fucα1-3)GlcNAcβ1-Hex (Robijn *et al*., unpublished results).
Figure 4 shows a MALDI-TOF MS (selected mass range) of the eluted fraction of a 114-4D12 affinity column loaded with pooled S. *mansoni* infection urine. The spectrum indicates the presence a series of fucosylated free oligosaccharides, H1N6F7 being the major species.
Figure 5 shows a MALDI-TOF MS (selected mass range) of the eluted fraction of a 114-4D12 affinity column loaded with pooled negative control urine. No significant signals were detected.
Figure 6 shows a series of MALDI-TOF MS (selected mass range) of the elution fraction of a 114-4D12 affinity column loaded with individual *S*. *mansoni* infection urines. For each urine (except #3987) the same set of MS signals (H1N6F7, major signal) was detected.
Figure 7 shows a representative example of a MALDI-TOF MS (selected mass range) of the eluted fraction of a 114-4D12 affinity column loaded with an individual Burundi negative control urine. No significant signals were detected.
Figure 8 shows a MALDI-TOF MS (selected mass range) of the eluted fraction of a 114-4D12 affinity column loaded with medium from *in vitro* incubated S. *mansoni* eggs. The spectrum shows that a similar series of structures is present (H1N6F7, major signal) as in the infection urines. In addition, in this preparation oligosaccharides lacking the hexose residue (in blue) are present.
Figure 9. Examples of oligosaccharide structural elements of **parasitic pathogens**
   A: *Leishmania donovani;* B,C,D: *Leishmania mexicana;* E,F: *Leishmania major;* G: *Haemonchus contortus;* H: *Acantocheilonema viteae;* I*, Onchocerca volvolus*
Figure 10. Examples of oligosaccharide structural elements of **bacterial pathogens**
   A: membrane derived oligosaccharide *Escherichia coli;* B,C,D: O-antigens LPS *E. coli;* E: capsular polysaccharide K1; F: capsular polysaccharide K5; G: capsular polysaccharide Group B *Streptococcus*

### References

Abdul-Salam, F. and Mansour, M.H. (2000). Identification and localization of a schistosome-associated fucosyllactose determinant expressed by Fasciola hepatica. Comp Immunol.Microbiol.Infect.Dis. 23, 99-111.
Argiro, L.L., Kolstadt, S.S., Henri, S.S., Dessein, H.H., Matabiau, V.V., Paris, P.P. Bourgois, A.A. and Dessein, A.J. (2000). Identification of a candidate vaccine peptide on the 37 kDa Schistosoma mansoni GAPDH. Vaccine 18, 2039-2048.
Basu, S., Das, K., and Basu, M. (2000). Glycosyltransferases in Glycosphingolipid Biosynthesis. In 'Carbohydrates in Chemistry and Biology'. B. Ernst, G.W. Hart, and P. SynayEds. pp. 329-343. Wiley-VCH, Weinheim.
Bergquist, N.R. (1992). Present aspects of immunodiagnosis of schistosomiasis. Mem.Inst.Oswaldo Cruz 87, 29-38.
Bergwerff, A.A., Van Dam, G.J. Rotmans, J.P., Deelder, A.M. Kamerling, J.P. and Vliegenthart, J.F. (1994). The immunologically reactive part of immunopurified circulating anodic antigen from Schistosoma mansoni is a threonine-linked polysaccharide consisting of ->6)-(beta-D-GlcpA-(1->3))-beta-D-GalpNAc-(1->repeating units. J.Biol.Chem. 269, 31510-31517.
Camacho, M., Alsford, S., and Agnew, A. (1996). Molecular forms of tegumental and muscle acetylcholinesterases of Schistosoma. Parasitology 112, 199-204.
Capron, A and Dessaint, J.P. (1992). Immunologic aspects of schistosomiasis. Annu.Rev.Med. 43, 209-218.
Chiang, C.P. and Caulfield, J.P. (1988). Schistosoma mansoni: ultrastructural demonstration of a miracidial glycocalyx that cross-reacts with antibodies raised against the cercarial glycocalyx. Exp.Parasitol. 67, 63-72.
De Jonge, N. Immunodiagnosis of Schistosoma infections by detection of the circulating anodic antigen. 1990. Leiden University. Thesis.
De Jonge, N., De Caluwe, P., Hilberath, G.W., Krijger, F.W., Polderman, A.M. and Deelder, A.M. (1989). Circulating anodic antigen levels in serum before and after chemotherapy with praziquantel in schistosomiasis mansoni. Trans.R.Soc.Trop.Med.Hyg. 83, 369-372.
De Jonge, N., Kremsner, P.G., Krijger, F.W., Schommer, G., Fillie, Y.E., Kornelis, D., Van Zeyl, R.J., Van Dam, G.J., Feldmeier, H. and Deelder, A.M. (1990). Detection of the schistosome circulating cathodic antigen by enzyme immunoassay using biotinylated monoclonal antibodies. Trans.R.Soc.Trop.Med.Hyg. 84, 815-818.
Deelder, A.M., De Jonge, N., Boerman, O.C., Fillie, Y.E., Hilberath, G.W., Rotmans, J.P., Gerritse, M.J., and Schut, D.W. (1989a). Sensitive determination of circulating anodic antigen in infected individuals by an enzyme-linked immunosorbent assay using monoclonal antibodies. Am.J.Trop.Med.Hyg. 40, 268-272.
Deelder, A.M., De Jonge, N., Fillie, Y.E., Kornelis, D., Helaha, D., Qian, Z.L., De Caluwe, P., and Polderman, A.M. (1989b). Quantitative determination of circulating antigens in human schistosomiasis mansoni using an indirect hemagglutination assay. Am.J.Trop.Med.Hyg. 40, 50-54.
Deelder, A.M., Qian, Z.L., Kremsner, P.G., Acosta, L., Rabello, A.L., Enyong, P., Simarro, P.P., Van Etten, E.C., Krijger, F.W., and Rotmans, J.P. (1994). Quantitative diagnosis of Schistosoma infections by measurement of circulating antigens in serum and urine. Trop.Geogr.Med. 46, 233-238.
Dell, A., Haslam, S.M., Morris, H.R., and Khoo, K.H. (1999). Immunogenic glycoconjugates implicated in parasitic nematode diseases. Biochim.Biophys.Acta 1455, 353-363.
Dunne, D.W., Butterworth, A.E., Fulford, A.J., Kariuki, H.C., Langley, J.G., Ouma, J.H., Capron, A., Pierce, R. and Sturrock, R.F. (1992). Immunity after treatment of human schistosomiasis: association between IgE antibodies to adult worm antigens and resistance to re-infection. European Journal of Immunology 1483-1494.
Eberl, M., Mountford, A.P., Jankovic, D. and Beck, E. (1999). Isolation of T-cell antigens by using a recombinant protein library and its application to the identification of novel vaccine candidates against schistosomiasis. Infect.Immun. 67, 3383-3389.
Engels, D., Chitsulo, L., Montresor, A. and Savioli, L. (2002). The global epidemiological situation of schistosomiasis and new approaches to control and research. Acta Trop. 82, 139-146.
Esko, J. (1999). Proteoglycans and glycosaminoglycans. In 'Essentials of Glycobiology'. A Varki, R.D. Cummings, J. Esko, H. Freeze, G.W. Hart, and J. Marth Eds. Gold Spring Harbor Laboratory Press; 145-159.
Esterre, P., Raobelison, A., Ramarokoto, C.E., Ravaoalimalala, V.E., Biosier, P. and Roux, J. (1998). Serum concentrations of sICAM-1, sE-, sP-, and sL-selectins in patients with Schistosoma mansoni infection and association with disease severity. Parasite Immunol. 20, 369-376.
Feldmeier, H. and Poggensee, G. (1993). Diagnostic techniques in schistosomiasis control. A review. Acta Trop. 52, 205-220.
Gryseels, B. (1989). The relevance of schistosomiasis for public health. Tropical medicine and parasitology, 134-142.
Gundersen, S.G., Ravn, J. and Haagensen, I. (1992). Early detection of circulating anodic antigen (CAA) in a case of acute schistosomiasis with Katayma fever. Scan.J.Infect.Dis. 549-552.
Hagan, P., Blumenthal, U.J., Dunn, D., Simpson, A.J. and Wilkins, H.A. (1991). Human IgE, IgG4 and resistance to re-infection with Schistosoma haematobium. Nature 349, 243-245.
Hall, T.M., Joseph, G.T., and Strand, M. (1995). Schistosoma mansoni: molecular cloning and sequencing of the 200-kDa chemotherapeutic target antigen. Exp.Parasitol. 80, 242-249.
Hamed, R.R., Maharem, T.M., and El Guindy, A.S. (1997). Proteoglycans from adult worms of Schistosoma haematobium. J.Helminthol. 71, 151-160.
Hart, G. W. (1999). Glycophospholipids Anchors. In 'Essentials of Glycobiology'. A. Varki, R.D. Cummings, J. Esko, H. Freeze, G.W. Hart, and J. Marth Eds. Gold Spring Harbor Laboratory Press; 131-143.
Haslam SM, Houston KM, Harnett W, Reason AJ, Morris HR, Dell A.(1999) Structural studies of N-glycans of filarial parasites. Conservation of phosphorylcholine-substituted glycans among species and discovery of novel chito-oligomers. J Biol Chem 274:20953-60.
Henle, K.J., Monson, T.P. and Stone, A. (1990). Enhanced glycosyltransferase activity during thermotolerance development in mammalian cells. J.Cell Physiol 142, 372-378.
Hokke, C.H. and Deelder, A.M. (2001). Schistosome glycoconjugates in host-parasite interplay. Glycoconj.J. 18, 573-587.
Hokke, C.H., Neeleman, A.P., Koeleman, C.A., and Van den Eijnden, D.H. (1998). Identification of an alpha2-fucosyltransferase and a novel alpha2-fucosyltransferase activity in cercariae of the schistosome Trichobilharzia ocellata: biosynthesis of the Fucalpha1-->2Fucalpha1-->3[Gal(NAc)beta1->4]GlcNAc sequence. Glycobiology 8, 393-406.
Hooker, C.W., Yang, W., Becker, M.M., Waine, G.J., Kalinna, B.H., Nimmo, K.A., Mcmanus, D.P. and Brindley, P.J. (1995). Schistosoma japonicum: heterogeneity in paramyosin genes. Acta Trop. 59, 131-141.
Horak P., Kovar L., Kolarova L., Nebesarova J. (1998). Cercaria-schistosomulum surface transformation of Trichobilharzia szidati and its putative immunological impact. Parasitology 116, 139-47.
Horak, P. and Kolarova, L. (2000). Survival of bird schistosomes in mammalian lungs. Int.J.Parasitol. 30, 65-68.
Huang, H.H., Tsai, P.L., and Khoo, K.H. (2001). Selective expression of different fucosylated epitopes on two distinct sets of Schistosoma mansoni cercarial O-glycans: identification of a novel core type and Lewis X structure. Glycobiology 11, 395-406.
Inal, J. and Bickle, Q. (1995). Sequence and immunogenicity of the 23-kDa transmembrane antigen of Schistosoma haematobium. Mol.Biochem.Parasitol. 74, 217-221.
Jacobs, W., Bogers, J.J., Timmermans, J.P., Deelder, A.M. and Van Marck, E.A. (1998). Adhesion molecules in intestinal Schistosoma mansoni infection. Parasitol.Res. 84, 276-280.
Jones, A.K., Bentley, G.N., Oliveros Parra, W.G., and Agnew, A. (2002). Molecular characterization of an acetylcholinesterase implicated in the regulation of glucose scavenging by the parasite Schistosoma. FASEB J. 16, 441-443.
Kagan, I.G. and Cahill, K.M. (1968). Parasitic serologic studies in Somaliland. Am.J.Trop.Med.Hyg. 17, 392-396.
Kahama, A.I. (1998). Detection of circulating soluble agg antigen (SEA) in human urinary schistosomiasis. Thesis.
Kahama, A.I., Ode, A.E., Kihara, R.W., Vennervald, B.J., Kombe, Y., Nkulila, T., C.F., Ouma, J.H. and Deelder, A.M. (1999). Urine circulating soluble egg antigen in relation to egg counts, hematuria, and urinary tract pathology before and after treatment in children infected with Schistosoma haematobium in kenya. Am.J.Trop.Med.Hyg. 61,215-219.
Kalinna, B.H. and McManus, D.P. (1997). A vaccine against the Asian schistosome. Schistosoma japonicum: an update on paramyosin as a target of protective immunity. Int.J.Parasitol. 27, 1213-1219.
Kamiya, H., Ozaki, T., Nakayama, H. and Inaba, T. (1993). Immunizing potential of ultraviolet-attenuated cercariae of Schistosoma mansoni in rodent hosts. Parasitol.Res. 79, 293-296.
Khoo, K.H., Chatterjee, D., Caulfield, J.P., Morris, H.R. and Dell, A. (1997b). Structural mapping of the glycans from the egg glycoproteins of Schistosoma mansoni and Schistosoma japonicum: identification of novel core structures and terminal sequences. Glycobiology 7, 663-677
Khoo, K.H., Chatterjee, D., Caulfield, J.P., Morris, H.R. and Dell, A. (1997a). Structural characterization of glycophingolipids from the eggs of Schistosoma mansoni and Schistosoma japonicum: identification of novel core structures and terminal sequences. Glycobiology 7, 663-677.
Khoo, K.H., Huang, H.H. and Lee, K.M. (2001). Characteristic structural features of schistosome cercarial N-glycans: expression of Lewis X and core xylosylation. Glycobiology 11, 149-163.
Khoo, K.H., Sarda, S., Xu, X., Caulfield, J.P., McNeil, M.R., Homans, S.W., Morris, H.R. and Dell, A. (1995). A unique multifucosylated -3GalNAc beta 1->4GlcNAc beta 1 -->3Gal alpha 1- motif constitutes the repeating unit of the complex O-glycans derived from the cercarial glycocalyx of Schistosoma mansoni. J.Biol.Chem. 270, 17114-17123.
Kindness, G., Long, W.F. and Williamson, F.B. (1980). Evidence for antithrombin III involvement in the anticoagulant activity of cellulose sulphate. Br.J.Pharmacol. 68, 645-649.
Klabunde, J., Berger, J., Jensenius, J.C. Klinkert, M.Q., Zelck, U.E., Kremsner, P.G. and Kun, J.F. (2000). Schistosoma mansoni: adhesion of mannan-binding lectin to surface glycoproteins of cercariae and adult worms. Exp.Parasitol. 95, 231-239.
Kolarova, L., Skirnisson, K. and Horak, P. (1999). Schistosome cercariae as the causative agent of swimmer's itch in Iceland. J.Heminthol. 73, 215-220.
Kornfeld, R. and Kornfeld, S. (1985). Assembly of asparagine-linked oligosaccharides. Annu.Rev.Biochem. 54, 631-664.
Koster, B. and Strand, M. (1994). Schistosoma mansoni: immunolocalization of two different fucose-containing carbohydrate epitopes. Parasitology 108, 433-446.
Lejoly-Boisseau, H., Appriou, M., Seigneur, M., Pruvost, A., Tribouley-Duret, J. and Tribouley J. (1999). Schistosoma mansoni: in vitro adhesion of parasite eggs to the vascular endothelium. Subsequent inhibition by a monoclonal antibody directed to a carbohydrate epitope. Exp.Parasitol. 91, 20-29.
Levery, S.B., Weiss, J.B., Salyan, M.E., Roberts, C.E., Hakomori, S., Magnani, J.L. and Strand, M. (1992). Characterization of a series of novel fucose-containing glycosphingolipid immunogens from eggs of Schistosoma mansoni. J.Biol.Chem. 267, 5542-5551.
Maddison, S.E. (1987). the present status of serodiagnosis and seroepidemiology of schistosomiasis. Diagn.Microbiol.Infect.Dis. 7, 93-105.
Maddison, S.E. (1991). Serodiagnosis of parasitic diseases. Clinical Microbiology Reviews 457-469.
Mansour, M.H. (1995). Evidence for a family of schistosome glycan-binding lectins in Biomphalaria alexandrina. Dev.Comp Immunol. 19, 365-376.
Mansour, M.H. (1996). Purification and characterization of SM 37: a fucosyllactose determinant-bearing glycoprotein probed by a Biomphalaria alexandrina lectin on adult male schistosomes. J.Parasitol. 82, 586-593.
McManus, D.P. (1999). The search for a vaccine against schistosomiasis-a difficult path but an achievable goal. Immunol.Rev. 171, 149-161.
McManus, D.P., Lui, S., Song, G., Xu, Y. and Wong J.M. (1998). The vaccine efficacy of native paramyosin (Sj-97) against Chinese Schistosoma japonicum. Int.J.Parasitol. 28, 1739-1742.
Morita, Y.S., Acosta-Serrano, A. and England, P.T. (2000). The biosynthesis of GPI Anchors. In 'Carbohydrates in Chemistry and Biology'. B. Ernst, G.W. Hart, and P. Sinay, Eds. Wiley-VCH, Weinheim, 417-433.
Nanduri, J., Dennis, J.E., Rosenberry, T.L., Mahmoud, A.A. and Tartakoff, A.M. (1991). Glycocalyx of bodies versus tails of Schistosoma mansoni cercariae. Lectin-binding, size, charge, and electron microscopic characterization. J.Biol.Chem. 266, 1341-1347.
Neeleman, A.P., Van der Knaap, W.P. and Van den Eijnden, D.H. (1994). Identification and characterization of a UDP-GalNAc:GlcNAc beta-R beta 1->4-N-acetylgalactosaminyl-transferase from cercariae of the schistosome Trichobilharzia ocellata. Catalysis of a key step in the synthesis of N,N'-diacetyllactosediamino (lacdiNAc)-type glycans. Glycobiology 4, 641-651.
Negm, H.I. (1996). Characterization of fucosyllactose determinant-bearing glycoproteins probed by a Biomphalaria alexandrina lectin in Schistosoma mansoni cercariae. Dev.Comp Immunol. 20, 87-96.
Nibbeling, H.A., Kahama, A.I., Van Zeyl, R.J., and Deelder, A.M. (1998a). Use of monoclonoal antibodies prepared against Schistosoma mansoni hatching fluid antigens for demonstration of Schistosoma haematobium circulating egg antigens in urine. Am.J.Trop.Med.Hyg. 58, 543-550.
Nibbeling, H.A., Van Lieshout, L. and Deelder, A.M. (1998b). Levels of circulating soluble egg antigen in urine of individuals infected with Schistosoma mansoni before and after treatment with praziquantel. Trans.R.Soc.Trop.Med.Hyg. 92, 675-677.
Nibbeling, H.A., Van Lieshout, L., Polman, K., Stelma, F.F., Polderman, A.M. and Deelder, A.M. (1998c). Serum circulating egg antigen levels in two areas endemic for Schistosoma mansoni. Trans.R.Soc.Trop.Med.Hyg. 92, 350-354.
Nourel Din, M.A., Nibbeling, R., Rotmans, J.P., Polderman, A.M., Krijger, F.W. and Deelder, A.M. (1994b). Quantitative determination of circulating soluble egg antigen in urine and serum of Schistosoma mansoni-infected individuals using a combined two-site enzyme-linked immunosorbent assay. Am.J.Trop.Med.Hyg. 50, 585-594.
Nutten, S., Papin, J.P., Woerly, G., Dunne, D.W., MacGregor, J., Trottein, F. and Capron, M. (1999). Selectin and Lewis(x) are required as co-receptors in antibody-dependent cell-mediated cytotoxicity of human eosinophils to Schistosoma mansoni schistosomula. Eur.J.Immunol. 29, 799-808.
Nyame A.K., Kawar Z.S., Cummings R.D. (2004). Antigenic glycans in parasitic infections: implications for vaccines and diagnostics. Arch.Biochem.Biophys 426, 182-200.
Nyame, K., Cummings, R.D. and Damian, R.T. (1988a). Characterization of the high mannose asparagine-linked oligosaccharides synthesized by Schistosoma mansoni adult male worms. Mol.Biochem.Parasitol. 28, 265-274.
Nyame, K., Cummings, R.D. and Damian, R.T. (1988b). Characterization of the N- and O-linked oligosaccharides in glycoproteins synthesized by Schistosoma mansoni schistosomula. J.Parasitol. 74, 562-572.
Nyame, K., Smith, D.F., Damian, R.T. and Cummings, R.D. (1989). Complex-type asparagine-linked oligosaccharides in glycoproteins synthesized by Schistosoma mansoni adult males contain terminal beta- linked N-acetylgalactosamine. J.Biol.Chem. 264, 3235-3243.
Paoletti L.C. and Kasper D.L. (2003). Glycoconjugate vaccines to prevent group B streptococcal infections. Expert.Opin.Biol.Ther. 3, 975-84.
Pearce, E.J. Magee, A.I., Smithers, S.R. and Simpson, A.J. (1991b). Sm25, a major schistosome tegumental glycoprotein, is dependent on palmitic acid for membrane attachment. EMBO J. 10, 2741-2746.
Pincet, F., Le Bouar, T., Zhang, U., Esnault, J., Mallet, J.M., Perez, E. and Sinay, P. (2001). Ultraweak sugar-sugar interactions for transient cell adhesion. Biophys.J. 80, 1354-1358.
Polderman, A.M. and De Caluwe, P. (1989). Eight years of targeted mass treatment of Schistosoma mansoni infection in Maniema, Zaire. Trop.Med.Parasitol. 40, 177-180.
Rabello, A. (1997). Diagnosing schistosomiasis. Mem.Inst.Oswaldo Cruz 92, 669-676.
Richter, D., Reynolds, S.R. and Harn. D.A. (1993). Candidate vaccine antigens that stimulate the cellular immune response of mice vaccinated with irradiated cercariae of Schistosoma mansoni. J.Immunol. 151, 256-265.
Riveau, G., Poulain-Godefroy, O.P., Dupre, L., Remoue, F., Mielcarek, N., Locht, C. and Capron, A. (1998). Glutathione S-transferases of 28kDa as major vaccine candidates against schistosomiasis. Mem.Inst.Oswaldo Cruz. 93, 87-94.
Rivera-Marrero, C.A. and Cummings, R.D. (1990). Schistosoma mansoni contains a galactosyltransferase activity distinct from that typically found in mammalian cells. Mol. Biochem.Parasitol. 43, 59-67.
Robertsen, N.P. and Cain, G.D. (1985). Isolation and characterization of glycosaminoglycans from Schistosoma mansoni. Comp Biochem.Physiol.B. 82, 299-306.
Sauma, S.Y. and Strand, M. (1990). Identification and characterization of glycosylphosphatidylinositol- linked Schistosoma mansoni adult worm immunogens. Mol.Biochem.Parasitol. 38, 199-209.
Sauma, S.Y., Tanaka, T.M. and Strand, M. (1991). Selective release of a glycosylphosphatidylinositol-anchored antigen from the surface of Schistosoma mansoni. Mol.Biochem.Parasitol. 46, 73-80.
Schachter, H. (1991). The 'yellow brick road' to branched complex N-glycans. Glycobiology 1, 453-461.
Schachter, H. (2000). The joys of HexNAc. The synthesis and function. Glycoconj.J. 17, 465-483.
Schachter, H. and Brockhausen, I. (1989). The biosynthesis of branched O-glycans. Symp.Soc.Exp.Biol. 43, 1-26.
Schmidt M.A., Riley L.W., Benz I. (2003). Sweet new world: glycoproteins in bacterial pathogens. Trends Microbiol. 11, 554-61.
Scot, J.C. and McManus, D.P. (2000). Molecular cloning and enzymatic expression of the 28-kDa gluthatione S-transferase of Schistosoma japonicum: evidence for sequence variation but lack of consistent accine efficacy in the murine host. Parasitol.Int. 289-300.
Secor, W.E., dos Reis, M.G., Ramos, E.A., Matos, E.P., Reis, E.A., do Carmo, T.M. and Harn, D.A., Jr. (1994). Soluble intercellular adhesion molecules in human schistosomiasis: correlations with disease severity and decreased responsiveness to egg antigens. Infect. Immun. 62, 5695-2701.
Shaker, Z.A., Hassanein, H.I., Hamed, R.R., Botros, S.S., Mahmoud, F.S., Mohamed, S.H., El Garem, A.A., De Jonge, N. and Deelder, A.M. (1992). Detection of circulating anodic antigen before and after specific chemotherapy in experimental murine Schistosomiasis mansoni. Int.J.Immunopharmacol. 14, 151-158.
Shi, Y.E., Jiang, C.F., Han, J.J., Li, Y.L. and Ruppel, A. (1990). Schistosoma japonicum: an ultraviolet-attenuated cercarial vaccine applicable in the field for water buffaloes. Exp.Parasitol. 71, 100-106.
Shuhua, X., Binggui, S., Chollet, J., Utzinger, J., and Tanner, M. (2000a). Tegumental changes in adult Schistosoma mansoni harbored in mice treated with arthemether. J.Parasitol. 86, 1125-1132.
Shuhua, X., Jiqing, Y., Jinying, M., Huifang, G., Peiying, J. and Tanner, M. (2000b). Effect of praziquantel together with artemether on Schistosoma japonicum parasites of different ages in rabbits. Parasitol.Int. 49, 25-30.
Shuhua, X., Utzinger, J., Chollet, J., Endriss, U., N'Goran, E.K. and Tanner, M. (2000c). Effect of artemether against Schistomosa haematobium in experimentally infected hamsters. Int.J.Parasitol. 30, 1001-1006.
Smith, M.A. and Clegg, J.A. (1984). Schistosoma mansoni: decay of resistance induced by gamma irradiated cercariae in the mouse. Trans.R.Soc.Trop.Med.Hyg. 78, 190-192.
Srivatsan, J., Smith, D.F. and Cummings, R.d. (1994). Demonstration of a novel UDPGalNAc:GlcNAc beta 1-4 N-acetylgalactosaminyltransferase in extracts of Schistosoma mansoni. J.Parasitol. 80, 884-890.
Stek, M. Fr, Minard, P., Dean, D.A. and Hall, J.E. (1981). Immunization of baboons with Schistosoma mansoni cercariae attenuated by gamma irradiation. Science 212 1518-1520.
Thomas P.G. and Harn D.A. Jr (2004). Immune biasing by helminth glycans. Cell Microbiol 6, 13-22
Trottein, F., Mollicone, R., Fontaine, J., de Mendonca, R., Piller, F., Pierce R., Oriol, R. and Capron, M. (2000). Molecular cloning of a putative alpha3-fucosyltransferase from Schistosoma mansoni. Mol.Biochem.Parasitol. 107, 279-287.
Trottein, F., Nutten, S., Angeli, V., Delerive, P., Teissier, E., Capron, A., Saels, B. and Capron, M. (1999). Schistosoma mansoni schistosomula reduce E-delectin and VCAM-1 expression in TNF-alpha-stimulated lung microvascular endothelial cells by interfering with the NF-kappaB pathway. Eur.J.Immunol. 29, 3691-3701.
Upreti R.K., Kumar M., Shankar V. (2003). Bacterial glycoproteins: functions, biosynthesis and applications. Proteomics 3, 363-79.
Van Dam, G.J. and Deelder, A.M. (1996). Glycoproteins of parasites. In 'Glycoproteins and Disease Amsterdam'. J. Montreuil, Vliegenthart J.F.G., and H. Schachter, Eds. Elsevier, Amsterdam, 159-182.
Van Dam, G.J., Bergwerff, A.A., Thomas-Oates, J.E., Rotmans, J.P., Kamerling J.P. Vliegenthart, J.F. and Deelder, A.M. (1994). The immunologically reactive O-linked polysaccharide chains derived from circulating cathodic antigen isolated from the human blood fluke Schistosoma mansoni have Lewis x as repeating unit. Eur.J.Biochem. 225, 467-482.
Van Dam, G.J., Kornelis, D., Van Zeyl, R.J., Rotmans, J.P. and Deelder, A.M. (1993a). Schistosoma mansoni: analysis of monoclonal antiboedies reactive with gut-associated antigens. Parasitol.Res. 79, 55-62.
Van de Wetering J.K., Van Remoortere A., Vaandrager A.B., Batenburg J.J., Van Golde, L.M., Hokke C.H., Van Hellemond J.J. (2004). Surfactant protein D binding to terminal alphal-3-linked fucose residues and to Schistosoma mansoni. Am.J.Respir Cell Mol.Biol. 31, 565-572.
Van den Berg T.K., Honing H., Franke N., Van Remoortere A., Schiphorst W.E., Liu F.T., Deelder A.M., Cummings R.D., Hokke C.H., Van Die I. (2004). LacdiNAc-glycans constitute a parasite pattern for galectin-3-mediated immune recognition. J.Immunol. 173, 1902-1907.
Van den Eijnden, D.H. (2000). On the origin of oligosaccharide species glycosyltransferase in action. In 'Carbohydrates in Chemistry and Biology'. B. Ernst, G.W. Hart, and P. Sinay, Eds. Wiley-VCH, Weinheim, 589-624.
Van den Eijnden, D.H., Bakker, H., Neeleman, A.P., Van den Nieuwenhof, I. and Van Die, I. (1997). Novel pathways in complex-type oligosaccharide synthesis: new vistas opened studies in invertebrates. Biochem.Soc.Trans. 25, 887-893.
Van der Kleij, D., Van Remoortere, A., Schuitemaker, J.N.H. Kapsenberg, M.L., Deelder, A.M., Tielens, A.G., Hokke, C.H. van Yazdanbakhsb, M. (2002). Triggering of innate immune responses by schistosome egg glycolipids and their carbohydrate epitope GalNAcβ1-4(Fucα1-2Fucα1-3)GlcNAc. J.Infect.Dis. 185, 531-539.
Van der Werf, M.J., De Vlas, S.J., Looman, C.W., Nagelkerke, N.J., Habbema, J.D. and Engels, D. (2002). Associating community prevalence of Schistosoma mansoni infection with prevalence of signs and symptoms. Acta Trop. 82, 127-137.
Van Lieshout, L., Polderman, A.M., Visser, L.G., Verwey, J.J. and Deelder, A.M. (1997). Detection of the circulating antigens CAA and CCA in a group of Dutch travellers with acute schistosomiasis. Trop.Med.Int.Health 2, 551-557.
Varki et al. (ed.) (1999): Essentials of Glycobiology, Chapters 21 and 36., Cold Spring Harbor Laboratory Press, New York
Varki, A. (1999a). Exploring the biological roles of glycans. In 'Essentials of Glycobiology'. A. Varki, R.D. Cummings, J. Esko, H. Freeze, G.W. Hart and J. Marth, Eds. Cold Spring Harbor Laboratory Press, 57-68.
Varki, A. (1999b). Glycosphingolipids. In 'Essentials of Glycobiology'. A. Varki, R.D. Cummings, J. Esko, H. Freeze, G.W. Hart and J. Marth, Eds. Cold Spring Harbor Laboratory Press, 155-129.
Visser, L.G., Polderman, A.M. and Stuiver, P.C. (1995). Outbreak of schistosomiasis among travelers returning from Mali, West Africa. Clin.Infect.Dis. 20, 280-285.
Willey, K.P. (1999). An elusive role for glycosylation in the structure and function of reproductive hormones. Hum.Reprod.Update. 5, 330-355.
Wuhrer, M., Dennis, R.D., Doenhoff, M.J., Lochnit, G. and Geyer, R. (2000). Schistosoma mansoni cercarial glycolipids are dominated by Lewis X and pseudo-Lewis Y structures. Glycobiology 10, 89-101.
Xiao, S.H., You, J.Q., Mei, J.Y., and Jiao, P.Y. (1994). Early treatment of schistosomal infection with artemether and praziquantel in rabbits. Chung Kuo Yao Li Hsueh Pao 15, 447-452.
Yang, W., Jackson, D.C., Zeng, Q. and McManus, D.P. (2000). Multi-epitope schistosome vaccine candidates tested for protective immunogenicity in mice. Vaccine 19, 103-113.
Zheng, Q., Van Die, I. and Cummings, R.D. (2002). Molecular cloning and characterization of a novel alpha 1,2- fucosyltransferase (CE2FT-1) from Caenorhabditis elegans. J.Biol.Chem. 277, 39823-39832.

## Claims

1. A method for analysing a sample comprising determining whether said sample comprises pathogen specific unconjugated oligosaccharide.

2. A method according to claim 1 wherein said pathogen specific unconjugated oligosaccharide comprises a structure of figure 9, 10 or table 1 or a functional part, derivative and/or analogue thereof.

3. A method according to claim 1 or claim 2, wherein said sample comprises a fluid obtained from an animal or plant.

4. A method according to claim 3, wherein said fluid comprises urine, blood, saliva, lymph fluid, cerebral spine fluid or synovial fluid.

5. A method according to any one of claims 1-4, wherein said sample is incubated with a binding molecule specific for a carbohydrate structure present on an oligosaccharide.

6. A method according to claim 5, further comprising determining whether said binding molecule comprises unconjugated oligosaccharide.

7. A method according to any one of claims 1-6, further comprising subjecting unconjugated oligosaccharide to a separation step.

8. A method according to claim 7, wherein said separation step separates oligosaccharides on the basis of size, mass, charge or polarity.

9. A method according to claim 8, wherein said separation step comprises electrophoresis and/or chromatography.

10. A method according to any one of claims 1-9, further comprising separating at least two unconjugated oligosaccharides by means of mass spectrometry.

11. A method according to any one of claims 1-10, wherein said pathogen is a microbe and/or a parasite.

12. A method according to claim 11, wherein said parasite is a helminth, preferably a schistosome.

13. A method according to any one of claims 5-12, wherein said binding molecule is specific for a structure of table 1.

14. A method according to any one of claims 1-13, further comprising typing said unconjugated oligosaccharide.

15. A method according to any one of claims 1-14, further comprising characterizing a structure of at least one of said separated unconjugated oligosaccharides.

16. A method according to any one of claims 2-15, further comprising providing said plant or animal with an oligosaccharide.

17. Use of unconjugated oligosaccharide in a sample obtained from an animal as a morbidity marker.

18. Use of unconjugated oligosaccharide in a sample obtained from an animal as a marker for the number of live helminth eggs in said animal.

19. Use of unconjugated oligosaccharides in urine as a marker for pathogen related disease or subclinical infection.
